# EUROPEAN PATENT APPLICATION

(11) **EP 1 604 817 A2**
(43) Date of publication of application: **14.12.2005**
(21) Application number: 05018298.9
(22) Date of filing: 16.09.2002
(51) Int. Cl.: B32B 31/00, B29C 43/22, B05C 1/08, B32B 5/04, A44B 18/00

(54) **Fastening tab articles with discrete elastic polymeric regions**

(30) Priority: 05.11.2001 US 13304
(62) Divisional of application: 02802766.2
(71) Applicant: 3M Innovative Properties Company, St. Paul, MN 55133-3427 (US)
(72) Inventor: The designation of the inventor has not yet been filed
(74) Representative: Vossius & Partner

(57) **Abstract**

Fastening tab articles including a substrate with one or more discrete polymeric regions (14) located thereon are disclosed. At least some of the discrete polymeric regions (14) are formed of an elastomeric thermoplastic composition that is transferred to the substrate in depressions formed on a transfer roll. The discrete elastomeric polymeric regions (14) can be used to provide elasticity to a substrate that is not elastic or they may be used to adjust the elasticity of the substrate that is itself elastic.

## Description

### FIELD OF THE INVENTION

The present invention relates to fastening tab articles and elastic articles that include one or more discrete polymeric regions of an elastomeric thermoplastic composition.

### BACKGROUND

The manufacture of articles that exhibit elasticity, i.e., the ability to at least partially recover their original shape after moderate elongation, may be desired for a number of reasons. For example, elasticity may be useful in connection with fastening systems for items such as garments (e.g., diapers, training pants, gowns, etc.). Elasticity in garments can provide what may be referred to as dynamic fit, i.e., the ability to stretch and recover in response to movement by the wearer.

Elasticity may also be useful in connection with other applications. For example, some fasteners may provide more consistent attachment if the fastener is held in tension that can be supplied by stretching the fastener and relying on the recovery forces to provide the desired tension. In other instances, elasticity may allow for easy adjustment of the size or length of a fastener or other article.

Although elasticity may be beneficial in a variety of different applications, it may raise issues in manufacturing. Many attempts to provide elasticity rely on separate elastic components that are, e.g., glued or sewn to a backing or other nonelastic member to provide the desired elasticity. The manufacture of such composite articles may be problematic in that secure attachment of the elastic components may be difficult to achieve and/or maintain. Further, the cost and difficulty of providing and attaching separate elastic components may be relatively high. The handling and attachment of separate elastic components can reduce throughput, cause additional waste (where the separate components are not securely attached), etc.

In other instances, an entire article may be constructed to provide the desired elasticity. For example, many elastic fastening systems rely on the use of elastic laminate backings in which the elastic materials are provided in the form of a film that is coextensive with the backing. Such an approach may add costs associated with providing a coextensive elastic layer or layers. Further, many elastic materials are not breathable. If the elastic laminate backings are to be used in garments, it may be desirable to perforate the backing to improve its breathability. Such additional processing does, however, add to the cost of producing the elastic laminate backing. Another potential disadvantage of elastic laminate backings is that it may be difficult to provide any variability in the elastic recovery forces generated in different portions of the backing.

### SUMMARY OF THE INVENTION

The present invention provides fastening tab articles and elastic articles including a substrate with one or more discrete polymeric regions located thereon. Each of the discrete polymeric regions is formed of elastomeric thermoplastic composition that is transferred to the substrate in depressions formed on a transfer roll. The discrete elastomeric polymeric regions can be used to provide elasticity to a substrate that is not elastic or they may be used to adjust the elasticity of a substrate that is itself elastic.

In other aspects, the present invention may provide substrates or articles that exhibit elasticity as a result of the addition of one or more discrete elastomeric polymeric regions, with the elasticity being provided in combination with discrete polymeric regions that may serve other functions, e.g., mechanical fasteners, stress distribution, bonding sites, etc.

One advantage of some articles of the present invention is the ability to transfer one or more discrete polymeric regions onto a major surface of a substrate, where the elastomeric thermoplastic material of the discrete polymeric region can be forced against the substrate by a transfer roll. If the substrate is porous, fibrous, etc., that pressure may enhance attachment of the discrete polymeric regions to the substrates by forcing a portion of the elastomeric thermoplastic composition to infiltrate the substrate and/or encapsulate fibers of the substrate.

Another advantage of the present invention is the ability to provide different thermoplastic compositions, such that some discrete polymeric regions may be formed of one thermoplastic composition, while other discrete polymeric regions are formed of a different thermoplastic composition. For example, discrete elastomeric polymeric regions may be provided on the same substrate as discrete nonelastomeric polymeric regions.

Another advantage of the present invention is the ability to control the shape, spacing, and volume of the discrete polymeric regions. This may be particularly advantageous because these parameters (shape, spacing, and volume) can be fixed regardless of the line speed of the system.

Another advantage of the present invention is the ability to provide one or more discrete polymeric regions that extend for the length of the substrate (while not being formed over the width of the substrate, i.e., the discrete polymeric regions are not coextensive with the major surface of the substrate).

Still another advantage of the articles of the present invention is the ability to provide one or more discrete polymeric regions on both major surfaces of a substrate. The discrete polymeric regions on the opposing major surfaces may be formed with the same or different materials and other characteristics as desired.

In another aspect, the present invention provides method for producing a composite web by providing a transfer roll with an exterior surface that includes one or more depressions formed therein; and delivering a molten elastomeric thermoplastic composition onto the exterior surface of the transfer roll. The method also includes wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one or more depressions after wiping the molten elastomeric thermoplastic composition from the exterior surface of the transfer roll; and transferring at least a portion of the molten elastomeric thermoplastic composition in the one or more depressions to a first major surface of a substrate by contacting the first major surface of the substrate to the exterior surface of the transfer roll and the molten elastomeric thermoplastic composition in the one or more depressions, followed by separating the substrate from the transfer roll, wherein one or more discrete polymeric regions formed of the elastomeric thermoplastic composition are located on the first major surface of the substrate after separating the substrate from the transfer roll.

In another aspect, the present invention provides method for producing a composite web by providing a transfer roll with an exterior surface that includes one or more depressions formed therein; and delivering a molten elastomeric thermoplastic composition onto the exterior surface of the transfer roll. The method also includes wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one or more depressions after wiping the molten elastomeric thermoplastic composition from the exterior surface of the transfer roll; and forcing a portion of a first major surface of a substrate into the one or more depressions, wherein the first major surface includes a porous surface including fibers, and wherein a portion of the elastomeric thermoplastic composition in the one or more depressions infiltrates the porous surface, and still further wherein the molten elastomeric thermoplastic composition encapsulates at least a portion of at least some of the fibers. The method also includes separating the substrate from the transfer roll, wherein one or more discrete polymeric regions formed of the elastomeric thermoplastic composition are located on the first major surface of the substrate after separating the substrate from the transfer roll.

In another aspect, the present invention provides a method for producing a composite web by providing a transfer roll with an exterior surface that includes one or more depressions formed therein; and delivering a molten elastomeric thermoplastic composition onto the exterior surface of the transfer roll. The method also includes wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one or more depressions after wiping the molten elastomeric thermoplastic composition from the exterior surface of the transfer roll; and transferring at least a portion of the molten elastomeric thermoplastic composition in the one or more depressions to a first major surface of a first substrate by contacting the first maj or surface of the first substrate to the exterior surface of the transfer roll and the molten elastomeric thermoplastic composition in the one or more depressions, followed by separating the first substrate from the transfer roll, wherein one or more discrete polymeric regions formed of the elastomeric thermoplastic composition are located on the first major surface of the first substrate after separating the first substrate from the transfer roll. The method further includes laminating a second substrate to the first major surface of the first substrate, wherein the one or more discrete polymeric regions on the first substrate are located between the first substrate and the second substrate after laminating the second substrate to the first substrate.

In another aspect, the present invention provides a method for producing a composite web by providing a first substrate including a first major surface and a second major surface, a plurality of discrete elastomeric polymeric regions formed of an elastomeric thermoplastic composition located on the first major surface of the first substrate, wherein each discrete elastomeric polymeric region of the plurality of discrete elastomeric polymeric regions infiltrates the first major surface of the first substrate. The method includes providing a second substrate having a first major surface and a second major surface, a plurality of discrete polymeric regions formed of a thermoplastic composition located on the first major surface of the second substrate, wherein each discrete polymeric region of the plurality of discrete polymeric regions infiltrates the first major surface of the second substrate. The method further includes laminating the first substrate to the second substrate.

In another aspect, the present invention provides an elastic fastening article including a substrate with first and second major surfaces; one or more mechanical fasteners attached to the first major surface of the substrate, wherein each mechanical fastener of the one or more mechanical fasteners includes a discrete thermoplastic region infiltrating the first major surface of the substrate, and wherein each mechanical fastener of the one or more mechanical fasteners further includes a plurality of fastening structures located thereon, the fastening structures facing away from the first major surface of the substrate. The article further includes one or more elastic elements attached to the substrate, wherein each elastic element of the one or more elastic elements includes a discrete elastomeric thermoplastic region infiltrating a portion of the substrate.

In another aspect, the present invention provides an elastic article including a substrate having first and second major surfaces; one or more elastic elements attached to the substrate, wherein each elastic element of the one or more elastic elements includes a discrete elastomeric thermoplastic region infiltrating a portion of the substrate; and one or more bonding sites located on the first major surface of the substrate.

In another aspect, the present invention provides an elastic article including a substrate having first and second major surfaces; one or more elastic elements attached to the substrate, wherein each elastic element of the one or more elastic elements includes a discrete elastomeric thermoplastic region infiltrating a portion of the substrate; and one or more slits formed through the substrate, wherein at least one of the one or more elastic elements spans each slit of the one or more slits.

In another aspect, the present invention provides an elastic article including a substrate with first and second major surfaces; one or more elastic elements attached to the substrate, wherein each elastic element of the one or more elastic elements includes a discrete elastomeric thermoplastic region infiltrating a portion of the substrate; and one or more pleats formed in the substrate, wherein at least one of the one or more elastic elements spans at least one pleat of the one or more pleats.

These and other features and advantages of the present invention are described below in connection with various illustrative embodiments of the invention.

### BRIEF DESCRIPTION OF THE DRAWINGS

FIG. 1 is a cross-sectional view of one composite web manufactured according to the methods of the present invention.
FIG. 2 is a perspective view of a pleated composite web manufactured according to the methods of the present invention.
FIG. 3 is a plan view of the pleated composite web of FIG. 2.
FIG. 4 is a perspective view of one polymer transfer process useful in providing discrete polymeric regions on a substrate in accordance with the methods of the present invention.
FIG. 4A illustrates another transfer roll and polymer source useful in connection with zoned delivery systems and methods
FIG. 4B is an enlarged partial cross-sectional view depicting wiping of the transfer roll by a doctor blade.
FIG. 4C is an enlarged partial cross-sectional view depicting a conformable backup roll forcing a substrate against a transfer roll.
FIG. 4D is an enlarged partial cross-sectional view depicting a mating backup roll including protrusions aligned with depressions in the transfer roll.
FIG. 5 is a plan view of a disposable diaper.
FIG. 6 is a plan view of one fastening tab manufactured from a portion of a composite web according to the present invention.
FIG. 7 is a cross-sectional view of the article of FIG. 6, taken along line 7-7 in FIG. 6.
FIG. 8 is a cross-sectional view of the article of FIG. 6, taken along line 8-8 in FIG. 6.
FIG. 9 is a perspective view of one system for manufacturing a composite web including discrete polymeric regions in accordance with the present invention.
FIG. 10 is a plan view of one composite web according to the present invention, the composite web including lines of separation.
FIG. 11 is a plan view of another fastening tab manufactured from a portion of a composite web according to the present invention.
FIG. 11A is a plan view of an elastic article manufactured from a composite web according to the present invention.
FIG. 11B is a plan view of an elastic article manufactured from a composite web according to the present invention.
FIG. 12 is a cross-sectional view of the article of FIG. 11, taken along line 12-12 in FIG. 11.
FIG. 13 is a cross-sectional view of the article of FIG. 11, taken along line 13-13 in FIG. 11.
FIG. 14 depicts one system for manufacturing a composite web including discrete polymeric regions in accordance with the present invention.
FIG. 15 is a plan view of one depression on a transfer roll that may be used in connection with the methods of the present invention.
FIG. 16 is a cross-sectional view of the depression of FIG. 15 taken along line 16-16 in FIG. 15.
FIG. 17 is a plan view of alternative depressions on a transfer roll that may be used in connection with the methods of the present invention.
FIG. 18 is a cross-sectional view of one depression of FIG. 17 taken along line 18-18 in FIG. 17.
FIG. 19 is a plan view of a portion of one composite web manufactured according to the present invention.
FIG. 20 is a perspective view of one transfer roll that may be used to manufacture the composite web of FIG. 19.
FIG. 21 is a plan view of a portion of one composite web manufactured according to the present invention that includes discrete polymeric regions extending across the width of the substrate.

### DETAILED DESCRIPTION OF ILLUSTRATIVE EMBODIMENTS OF THE INVENTION

The present invention provides methods and systems for producing composite webs that include a substrate with discrete elastomeric polymeric regions located one and/or within the substrate. Various different constructions will now be described to illustrate various embodiments of the composite webs that can be manufactured in accordance with the methods of the present invention. These illustrative constructions should not be considered to limit the present invention, which is to be limited only by the claims that follow.

For example, some embodiments of the invention will be described in the context of a disposable absorbent article, such as a disposable diaper. It is, however, readily apparent that the present invention could also be employed with other articles, such as caps, gowns, shoe covers, feminine care articles, incontinence garments and the like.

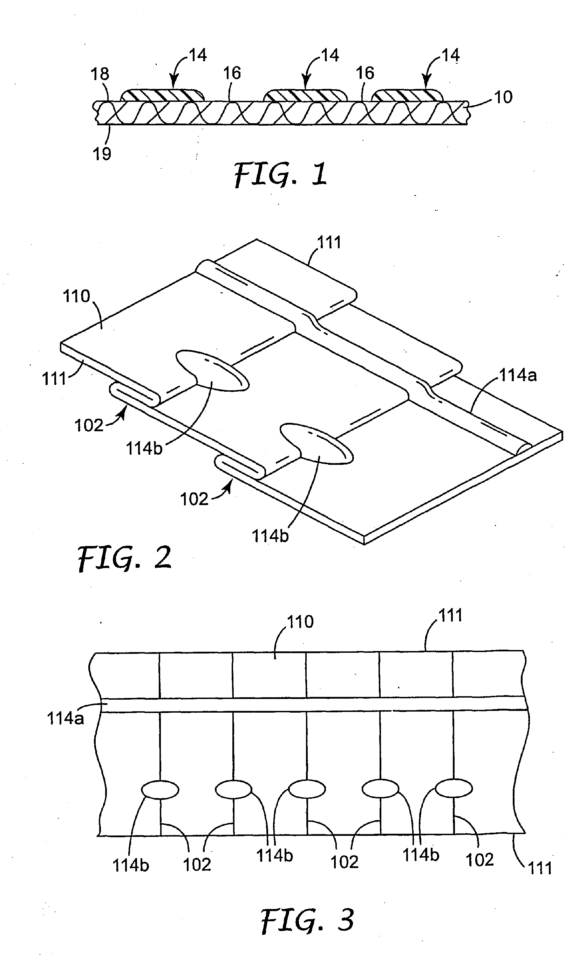

FIG. 1 is a cross-sectional view of a portion of one composite web manufactured in accordance with the present invention. The composite web includes a substrate 10 with a first major surface 18 and a second major surface 19. A plurality of discrete polymeric regions 14 are located on the first major surface 18 of the substrate 10. The regions 14 may preferably be formed of an elastomeric thermoplastic composition as discussed in more detail below.

The different discrete polymeric regions 14 are separated by exposed areas 16 on the first major surface 18 of substrate 10. As depicted in FIG. 1, the spacing, i.e., the size of the exposed area 16 between the discrete polymeric regions 14 may be the same or different. For example, the exposed area 16 located between the left-most pair of discrete polymeric regions 14 is larger than the exposed area 16 located between the right-most pair of discrete polymeric regions 14.

The discrete polymeric regions 14 may cover any desired portion of the surface area of the substrate 10 on which they are positioned, although it will be understood that the discrete polymeric regions 14 will not cover all of the surface of the substrate 10. Some variations in the percentage of surface area occupied by discrete polymeric regions may be as described in, for example, pending U.S. Patent Application Serial No. 09/257,447, entitled WEB HAVING DISCRETE STEM REGIONS, filed on Feb. 25, 1999 (published as International Publication No. WO 00/50229).

Further, although the discrete polymeric regions 14 are depicted as being disconnected from each other, it should be understood that some composite webs manufactured with the systems and methods of the present invention may include a relatively thin skin layer of the thermoplastic composition used to form the discrete polymeric regions. Such a skin layer may, in some instances, connect some or all of the discrete polymeric regions on the composite web. Where, e.g., the skin layer is formed of an elastomeric thermoplastic composition, the amount of polymeric material in the skin layer will, however, be insufficient to significantly affect elasticity of the substrate 10 outside of the thicker discrete polymeric regions 14.

The substrates used in connection with the composite webs of the present invention may have a variety of constructions. For example, the substrates may be a woven material, nonwoven material, knit material, paper, film, or any other continuous media that can be fed through a nip point. The substrates may have a wide variety of properties, such as extensibility, elasticity, flexibility, conformability, breathability, porosity, stiffness, etc. Further, the substrates may include pleats, corrugations or other deformations from a flat planar sheet configuration.

In some instances, the substrates may exhibit some level of extensibility and also, in some instances, elasticity. Extensible webs that may be preferred may have an initial yield tensile force of at least about 50 gm/cm, preferably at least about 100 gm/cm. Further, the extensible webs may preferably be extensible nonwoven webs.

Suitable processes for making a nonwoven web that may be used in connection with the present invention include, but are not limited to, airlaying, spunbond, spunlace, bonded melt blown webs and bonded carded web formation processes. Spunbond nonwoven webs are made by extruding a molten thermoplastic, as filaments from a series of fine die orifices in a spinneret. The diameter of the extruded filaments is rapidly reduced under tension by, for example, by non-eductive or eductive fluid-drawing or other known spunbond mechanisms, such as described in U.S. Patent Nos. 4, 340,563 (Appel et al.); 3,692,618 (Dorschner et al.); 3,338,992 and 3,341,394 (Kinney); 3,276,944 (Levy); 3,502,538 (Peterson); 3,502,763 (Hartman) and 3,542,615 (Dobo et al.). The spunbond web is preferably bonded (point or continuous bonding).

The nonwoven web layer may also be made from bonded carded webs. Carded webs are made from separated staple fibers, which fibers are sent through a combing or carding unit which separates and aligns the staple fibers in the machine direction so as to form a generally machine direction-oriented fibrous nonwoven web. However, randomizers can be used to reduce this machine direction orientation.

Once the carded web has been formed, it is then bonded by one or more of several bonding methods to give it suitable tensile properties. One bonding method is powder bonding wherein a powdered adhesive is distributed through the web and then activated, usually by heating the web and adhesive with hot air. Another bonding method is pattern bonding wherein heated calender rolls or ultrasonic bonding equipment are used to bond the fibers together, usually in a localized bond pattern though the web can be bonded across its entire surface if so desired. Generally, the more the fibers of a web are bonded together, the greater the nonwoven web tensile properties.

Airlaying is another process by which fibrous nonwoven webs useful in the present invention can be made. In the airlaying process, bundles of small fibers usually having lengths ranging between about 6 to about 19 millimeters are separated and entrained in an air supply and then deposited onto a forming screen, often with the assistance of a vacuum supply. The randomly deposited fibers are then bonded to one another using, for example, hot air or a spray adhesive.

Meltblown nonwoven webs may be formed by extrusion of thermoplastic polymers from multiple die orifices, which polymer melt streams are immediately attenuated by hot high velocity air or steam along two faces of the die immediately at the location where the polymer exits from the die orifices. The resulting fibers are entangled into a coherent web in the resulting turbulent airstream prior to collection on a collecting surface. Generally, to provide sufficient integrity and strength for the present invention, meltblown webs must be further bonded such as by through air bonding, heat or ultrasonic bonding as described above.

A web can be made extensible by skip slitting as is disclosed in, e.g., International Publication No. WO 96/10481 (Abuto et al.). If an elastic, extensible web is desired, the slits are discontinuous and are generally cut on the web prior to the web being attached to any elastic component. Although more difficult, it is also possible to create slits in the nonelastic web layer after the nonelastic web is laminated to the elastic web. At least a portion of the slits in the nonelastic web should be generally perpendicular (or have a substantial perpendicular vector) to the intended direction of extensibility or elasticity (the at least first direction) of the elastic web layer. By generally perpendicular it is meant that the angle between the longitudinal axis of the chosen slit or slits and the direction of extensibility is between 60 and 120 degrees. A sufficient number of the described slits are generally perpendicular such that the overall laminate is elastic. The provision of slits in two directions is advantageous when the elastic laminate is intended to be elastic in at least two different directions.

A nonwoven web used in connection with the present invention can also be a necked or reversibly necked nonwoven web as described in U.S. Patent Nos. 4,965,122; 4,981,747; 5,114,781; 5,116,662; and 5,226,992 (all to Morman). In these embodiments the nonwoven web is elongated in a direction perpendicular to the desired direction of extensibility. When the nonwoven web is set in this elongated condition, it will have stretch and recovery properties in the direction of extensibility.

The substrates used in connection with the present invention may preferably exhibit some porosity on one or both of the major surfaces of the substrate such that when a molten thermoplastic composition is provided on one of the major surfaces of the substrate, a mechanical bond is formed between the molten thermoplastic composition and the substrate as the molten thermoplastic composition infiltrates and/or encapsulates a portion of the porous surface of the substrate. As used in connection with the present invention, the term "porous" includes both structures that include voids formed therein, as well as structures formed of a collection of fibers (e.g., woven, nonwoven, knit, etc.) that allow for the infiltration of molten thermoplastic composition into the interstices between fibers. If the porous surface includes fibers, the thermoplastic composition may preferably encapsulate fibers or portions of fibers on the surface of the substrate.

As used herein, the term "fiber" includes fibers of indefinite length (e.g., filaments) and fibers of discrete length, e.g., staple fibers. The fibers used in connection with the present invention may be multicomponent fibers. The term "multicomponent fiber" refers to a fiber having at least two distinct longitudinally coextensive structured polymer domains in the fiber cross-section, as opposed to blends where the domains tend to be dispersed, random, or unstructured. The distinct domains may thus be formed of polymers from different polymer classes (e.g., nylon and polypropylene) or be formed of polymers from the same polymer class (e.g., nylon) but which differ in their properties or characteristics. The term "multicomponent fiber" is thus intended to include, but is not limited to, concentric and eccentric sheath-core fiber structures, symmetric and asymmetric side-by-side fiber structures, island-in-sea fiber structures, pie wedge fiber structures, and hollow fibers of these configurations.

The type and construction of the material or materials in the substrate should be considered when selecting an appropriate substrate to which a molten thermoplastic composition is applied. Generally, such materials are of the type and construction that do not melt, soften, or otherwise disintegrate under the temperatures and pressures experienced during the step of transferring the thermoplastic composition to the substrate. For example, the substrate should have sufficient internal strength such that it does not fall apart during the process. Preferably, the substrate has sufficient strength in the machine direction at the temperature of the transfer roll to remove it intact from the transfer roll.

Although the substrates depicted in the various cross-sectional views of the present invention are illustrated as single layer structures, it should be understood that the substrates may be of single or multi-layer construction. If a multi-layer construction is used, it will be understood that the various layers may have the same or different properties, constructions, etc. Some of these variations may be as described in, for example, pending U.S. Patent Application Serial No. 09/257,447, entitled WEB HAVING DISCRETE STEM REGIONS, filed on Feb. 25, 1999 (published as International Publication No. WO 00/50229).

The discrete polymeric regions 14 may be formed of a wide variety of different thermoplastic polymeric materials. The thermoplastic compositions used in connection with the methods of the present invention should be capable of flowing or entering into depressions formed in a polymer transfer roll as will be described below. Furthermore, it may be desirable that some of the thermoplastic compositions also exhibit a relatively high degree of moldability, i.e., the ability to enter and preferably take the shape of a cavity when subjected to the proper conditions of temperature and pressure.

Suitable thermoplastic compositions are those that are melt processable. Such polymers are those that will flow sufficiently to at least partially fill the depressions, yet not significantly degrade during a melt process. A wide variety of thermoplastic compositions have suitable melt and flow characteristics for use in the process of the present invention depending on the geometry of the depressions and the processing conditions. It may further be preferred that the melt processable materials and conditions of processing are selected such that any viscoelastic recovery properties of the thermoplastic composition do not cause it to significantly withdraw from the wall(s) of the depressions until transfer of the thermoplastic composition to a substrate is desired.

As used in connection with the present invention, "thermoplastic" (and variations thereof) means a polymer or polymeric composition that softens when exposed to heat and returns to its original condition or near its original condition when cooled to room temperature.

Some examples of thermoplastic compositions that may be used in connection with the present invention include, but are not limited to, polyurethanes, polyolefins (e.g., polypropylenes, polyethylenes, etc.), polystyrenes, polycarbonates, polyesters, polymethacrylates, ethylene vinyl acetate copolymers, ethylene vinyl alcohol copolymers, polyvinylchlorides, acrylate modified ethylene vinyl acetate polymers, ethylene acrylic acid copolymers, nylons, fluorocarbons, etc. These materials can be elastomeric or nonelastomeric (e.g., polycarbonates, polymethacrylates, and polyvinylchlorides).

At least one or more of the discrete polymeric regions formed on a substrates in connection with the composite webs of the present invention is formed of an elastomeric thermoplastic composition. An elastomeric thermoplastic composition is a polymeric composition that melts and returns to its original condition or near its original condition upon cooling and exhibits elastomeric properties at ambient conditions (e.g., room temperature and pressure). As used in connection with the present invention, "elastomeric" means that the material will substantially resume its original shape after being stretched. Further, the elastomeric materials may preferably sustain only small permanent set following deformation and relaxation, which set is preferably no greater than about 30 percent and more preferably no greater than about 20 percent of the original length at moderate elongation, e.g., about 50%. The elastomeric materials can be both pure elastomers and blends with an elastomeric phase or content that will still exhibit substantial elastomeric properties at room temperature. U.S. Patent No. 5,501,679 (Krueger et al.) provides some further discussion regarding elastomeric materials that may be considered for use in connection with the present invention.

The elastomeric thermoplastic compositions can include one or more polymers. For example, the elastomeric thermoplastic composition could be a blend with an elastomeric phase such that the composition exhibits elastomeric properties at room temperature. Suitable elastic thermoplastic polymers include block copolymers such as conventional A-B or A-B-A block copolymers (e.g., styrene-isoprene-styrene, styrene-butadiene-styrene, styrene-ethylene-butylene-styrene block copolymers), elastomeric polyurethanes, olefinic elastomers, particularly elastomeric ethylene copolymers (e.g., ethylene vinyl acetates, ethylene/octene copolymer elastomers, ethylene/propylene/diene terpolymer elastomers), as well as mixtures of these with each other, with other elastomeric thermoplastic polymers, or with nonelastomeric thermoplastic polymers.

The thermoplastic compositions used in connection with the present invention can also be combined with various additives for desired effect. These include, for example, fillers, viscosity reducing agents, plasticizers, tackifiers, colorants (e.g., dyes or pigments), antioxidants, antistatic agents, bonding aids, antiblocking agents, slip agents, stabilizers (e.g., thermal and ultraviolet), foaming agents, microspheres, glass bubbles, reinforcing fibers (e.g., microfibers), internal release agents, thermally conductive particles, electrically conductive particles, and the like. The amounts of such materials that can be useful in the thermoplastic compositions can be readily determined by those skilled in the art of processing and using such materials.

FIGS. 2 and 3 depict another embodiment of a composite web manufactured in accordance with the present invention that includes a substrate 110 on which a plurality of discrete polymeric regions 114a and 114b are located. The substrate 110 includes pleats 102 that extend across the width of the substrate 110 (where the width is defined by the opposing edges 111 of the substrate 110).

Discrete polymeric region 114a is an example of a discrete polymeric region that extends along the length of the substrate 110, such that the discrete polymeric region 114a spans multiple pleats 102 as seen in FIGS. 2 and 3. Discrete polymeric regions 114b are examples of smaller discrete polymeric regions that span only one pleat 102 formed in the substrate 110.

When the discrete polymeric regions 114a arid/or 114b are formed of an elastomeric thermoplastic composition, they may act to prevent unfolding of the pleats 102 or they may attempt to restore the pleats to their folded state if the substrate 110 is stretched in a manner that would cause the pleats 102 to unfold.

The smaller discrete polymeric regions 114b are oval in shape, but it will be understood that the discrete polymeric regions could be provided in any desired shape, e.g., squares, rectangles, hexagons, etc. The shapes may or may not be in the form of recognized geometric shapes, but may be randomly formed with irregular perimeters. In addition, the shapes may not necessarily be solid figures, but may include voids formed within the shape in which none of the thermoplastic composition is transferred. In yet another alternative, some or all of the discrete polymeric regions may be in the form of indicia, i.e., letters, numbers, or other graphic symbols.

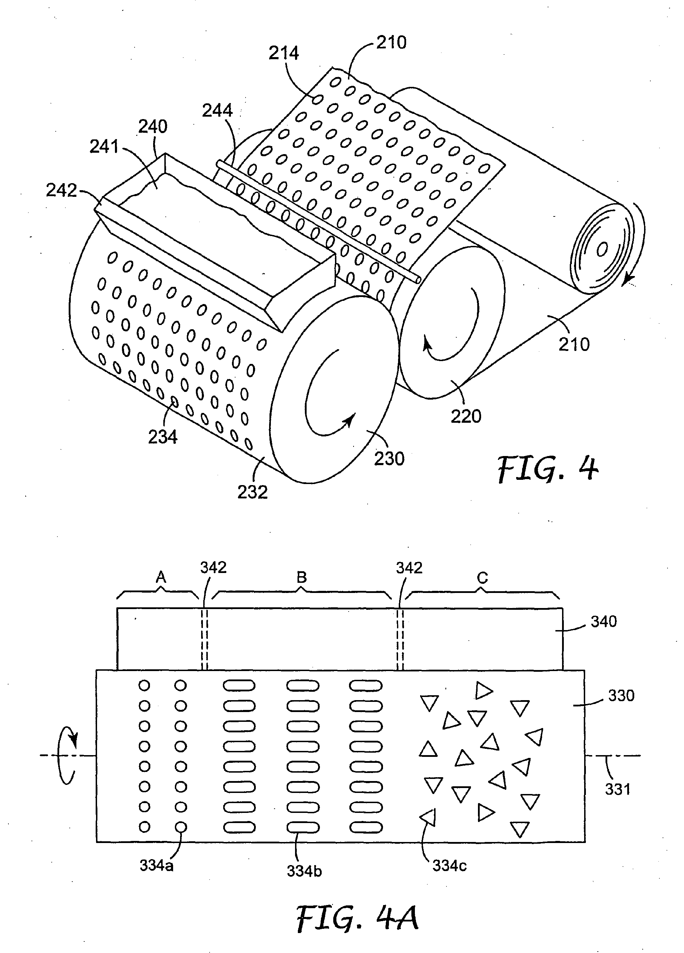

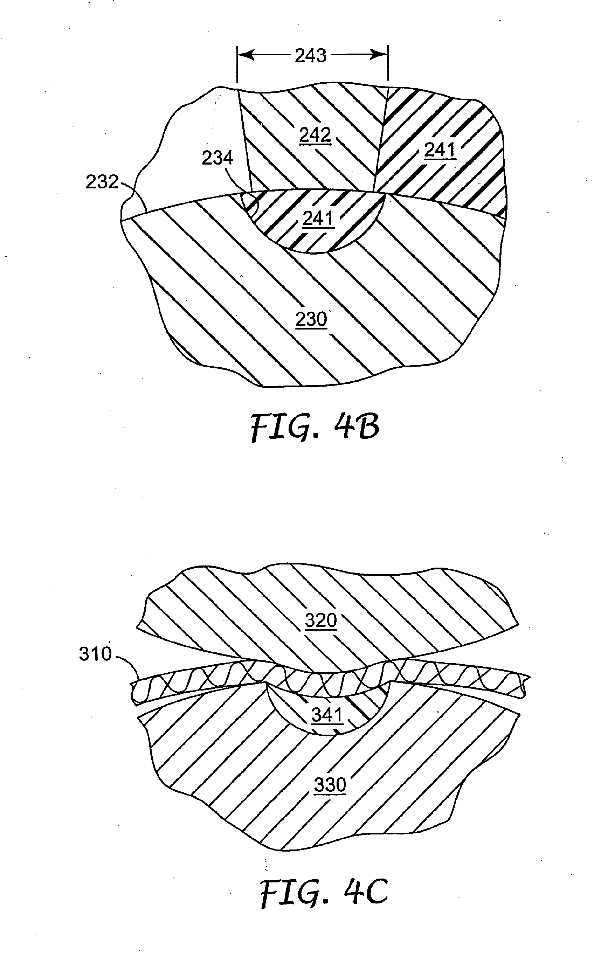

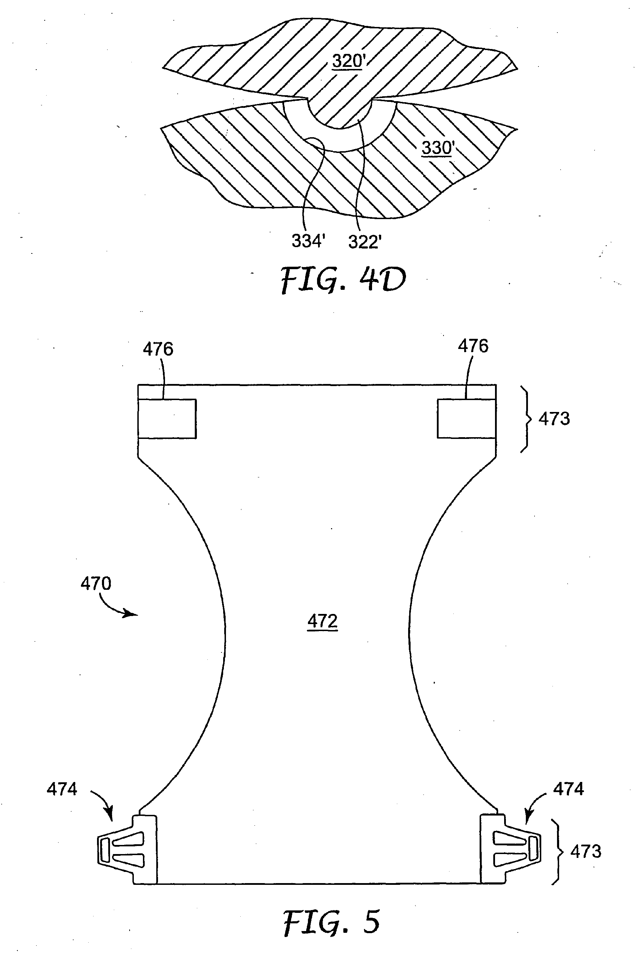

FIG. 4 is a perspective view of one system and method of providing discrete polymeric regions on one surface of a substrate 210 in accordance with the principles of the present invention. The system depicted in FIG. 4 includes a substrate 210 that defines a web path through the system. The substrate 210 moves through the system in a downstream direction indicated by the rotation arrows on the various rolls. After being unwound or otherwise provided from a supply (e.g., the substrate 210 may be manufactured in-line with the system depicted in FIG. 4), the substrate 210 is directed into a transfer nip formed between a backup roll 220 and a transfer roll 230.

The process of providing discrete polymeric regions on the substrate 210 includes delivering a supply of a molten thermoplastic composition to the exterior surface 232 of transfer roll 230 that includes a one or more depressions 234 formed in its exterior surface 232. The molten thermoplastic composition 241 is supplied to the exterior surface 232 of the transfer roll 230 by a delivery apparatus in the form of a trough 240 (or other supply apparatus, e.g., extruder, gear pump, etc.). The excess molten thermoplastic composition is wiped or removed from the exterior surface 232 by a doctor blade 242 acting against the exterior surface 232 of the transfer roll 230. Although it may be ideal to remove all of the thermoplastic composition from the exterior surface 232 of the transfer roll 230, some of the thermoplastic composition may remain on the exterior surface 232 after wiping by the doctor blade 242.

The depressions 234 formed in the exterior surface 232 of the transfer roll 230 preferably receive a portion of the molten thermoplastic composition when the molten thermoplastic composition is deposited on the exterior surface 232 of the transfer roll 230. If the depressions 234 are not completely filled during or by the deposition of molten thermoplastic composition, the wiping action of the doctor blade 242 on the exterior surface 232 of the transfer roll 230 may assist in substantially filling the depressions with molten thermoplastic composition.

Although FIG. 4 depicts the application of only one thermoplastic composition using the transfer roll 230, it will be understood that two or more different thermoplastic compositions may be applied to the exterior surface of the transfer roll 230. FIG. 4A depicts a portion of one system in which a trough 340 is used to deliver three molten thermoplastic compositions (in zones A, B, & C) to the surface of a transfer roll 330 that rotates about an axis 331. The trough 340 may, for example, include barriers 342 such that molten thermoplastic compositions in the different zones of the trough 340 do not mix during processing. In another alternative, separate and distinct troughs could be used for each different thermoplastic composition to be applied to the transfer roll 330. The troughs or zones may, e.g., be used to deliver elastomeric and nonelastomeric thermoplastic compositions to the roll 330 at the same time.

The transfer roll 330 also includes different sets of depressions 334a, 334b, and 334c over which the different molten thermoplastic compositions may be applied. The depressions in the different zones on transfer roll 330 are differently shaped, have different sizes, and have different spacings. For example, the triangular depressions in zone C are arranged in an irregular, non-repeating pattern while the depressions in zones A & B are arranged in regular, repeating patterns.

With the system of FIG. 4A, different sets of discrete polymeric regions may be formed on a single substrate using different thermoplastic compositions. As a result, the thermoplastic compositions may be selected for any of a number of different properties related to manufacturing or end-use performance of the finished articles made using the composite webs.

Control over the temperatures of the various rolls in the system depicted in FIG. 4 may be useful in obtaining the desired products. It may be preferred, e.g., that the exterior surface 232 of the transfer roll 230 be heated to a selected temperature that is at or above the melt temperature of the thermoplastic composition to be transferred to the substrate 210. Heating the transfer roll 230 may also enhance filling of the depressions 234 by the molten thermoplastic composition.

Because the molten thermoplastic composition 241 is itself heated within the trough 240, the doctor blade 242 will typically be heated by the molten thermoplastic composition. It may alternatively be desirable to control the temperature of the doctor blade 242 separately from the trough 240 containing the molten thermoplastic composition 241. For example, it may be desirable to heat the doctor blade 242 to a temperature above the melt temperature of the molten thermoplastic composition.

FIG. 4B is an enlarged partial cross-sectional view depicting one relationship between a doctor blade 242 and depression 234 in a transfer roll 230. Another characteristic of the doctor blade 242 that may be controlled is its thickness or length 243 along the exterior surface of the transfer roll 230 (as measured in the machine direction or the direction of rotation of the transfer roll). For example, a thicker or longer doctor blade 242 may help by allowing the molten thermoplastic composition more time to relax within the depressions 234, thereby improving filling of the depressions. In addition to varying the length of the doctor blade 242, the pressure or force exerted on the transfer roll 230 by the doctor blade 242 may also be adjusted based on a variety of factors including, e.g., the characteristics of the molten thermoplastic composition, the transfer roll characteristics, etc.

With the depressions 234 at least partially filled with the desired molten thermoplastic composition, the transfer roll 230 continues to rotate until the depressions 234 and the molten thermoplastic composition they contain are forced into contact with the substrate 210 against backup roll 220 at the transfer nip (i.e., the nip formed by the transfer roll 230 and the backup roll 220). It is at this point that transfer of the molten thermoplastic composition in the depressions 234 to the substrate 210 begins. It should be understood that under certain conditions, only a portion of the thermoplastic composition in the depressions 234 may transfer to the substrate 210.

When a substrate 210 that includes one or more porous major surfaces on which the molten thermoplastic composition is deposited is used in connection with the methods of the present invention, a mechanical bond is preferably formed by infiltration of the molten thermoplastic composition into the porous surface of the substrate 210. As used in connection with the present invention, the term "porous" includes both structures that include voids formed therein, as well as structures formed of a collection of fibers (e.g., woven, nonwoven, or knit) that allow for the infiltration of molten thermoplastic compositions.

The nip pressure between the transfer roll 230 and the backup roll 220 is preferably sufficient such that a portion of the thermoplastic composition in the discrete polymeric regions infiltrates into and/or encapsulates a portion of the porous substrate 210 to improve attachment of the discrete polymeric regions to the substrate 210. Where the surface of the substrate 210 includes fibers (e.g., where the substrate 210 includes woven, nonwoven, or knit materials on its major surfaces), it may be preferred that the thermoplastic composition encapsulate all or a portion of at least some of the fibers on the surface of the substrate 210 to improve attachment of the discrete polymeric regions to the substrate 210.

Under some conditions the molten thermoplastic composition in the depressions 234 may completely permeate the substrate 210 if, e.g., the substrate 210 is porous throughout its thickness. In other instances, penetration of the molten thermoplastic composition may be limited to the outer layer or layers of the substrate 210.

It should, however, be understood that although the outer surfaces of the substrate 210 may exhibit some porosity, that porosity may not necessarily extend through the entire thickness of the substrate 210. For example, the substrate 210 may have a variety of different layers, with one of the layers being substantially non-porous. In another alternative, the overall thickness of the substrate 210 may render it non-porous as a whole, even though the outer surfaces of the substrate 210 exhibit some porosity as discussed above.

The backup roll 220 may possess a variety of different characteristics depending on the types of substrate materials and/or molten thermoplastic compositions being processed. In some instances, the exterior of the backup roll 220 may be a rubber or other conformable material that conforms to the shape of the transfer roll 230. If a conformable material such as rubber is used, it may, e.g., have a durometer of, e.g., about 10-90 Shore A.

One such variation at the transfer nip is depicted in FIG. 4C, in which a conformable backup roll 330 is depicted as forcing a portion of the substrate 310 into the depression 334 (and the thermoplastic composition 341 contained therein). If the surface of the substrate 310 facing the depression 334 is porous, a portion of the molten thermoplastic composition 341 may be forced into or infiltrate the porous surface of the substrate 310. Forcing the substrate 310 into the depression may be particularly beneficial if the depression 334 is not completely filled with the molten thermoplastic composition 341 to improve the likelihood of contact between the substrate 310 and the molten thermoplastic composition 341.

Alternatively, the surface of the substrate may be forced into the depressions on the transfer roll using a mating backup roll. This variation at the transfer nip is depicted in FIG. 4D in which the backup roll 320' includes protrusions 322' that are complementary to or mate with the depressions 334' on the transfer roll 330'. The protrusions 322' would preferably force a substrate into the depressions with the same results and benefits described above with respect to FIG. 4C. A mating backup roll 320' could be formed of any suitable conformable material, nonconformable material, or combination of conformable or nonconformable materials.

Heating or otherwise controlling the temperature of the transfer roll is discussed above. It should also be appreciated that the temperature of the exterior surface of the backup roll may be controlled. For example, it may be desirable to cool the surface of the backup roll to a selected temperature below the temperature of the transfer roll. Cooling of the backup roll may be beneficial in maintaining the integrity of the substrate, particularly if the substrate integrity can be degraded from the heat of the transfer roll (if the transfer roll is heated) and/or the molten thermoplastic composition in the depressions of the transfer roll.

After passing through the transfer nip formed between the backup roll 220 and the transfer roll 230, the substrate 210 continues around the backup roll 220 as seen in FIG. 4. In some instances, a portion of the molten thermoplastic composition in the depressions may remain in the depressions 234 while the substrate 210 is pulled away from the transfer roll 230. As a result, the molten thermoplastic composition in the depressions 234 may tend to elongate or string between the depressions in transfer roll 230 and the substrate 210.

A device, such as a hot wire 244 seen in FIG. 4, may be used to sever any strands of thermoplastic composition that may be formed as the substrate 210 separates from the transfer roll 230. Other devices and/or techniques may be used to accomplish the desired severing of any molten thermoplastic composition strands. Examples may include, but are not limited to hot air knives, lasers, etc. Furthermore, under certain conditions, stringing of the thermoplastic composition may not be encountered during manufacturing.

The tendency of the molten thermoplastic composition in the depressions 234 to string as the substrate exits the transfer nip also raises another issue that should be considered when developing processes according to the present invention. That issue is the internal cohesive strength of the substrate 210 and/or the tensile strength of the substrate 210. This issue may be of more concern if the substrate 210 includes a fibrous construction (e.g., woven, nonwoven, or knit fibers) that could be separated from the remainder of the substrate by the forces exerted when the substrate 210 is pulled away from the transfer roll 230. These considerations may be more important if the molten thermoplastic composition has properties (e.g., tackiness, tensile strength, etc.) such that strands of the molten thermoplastic composition can exert forces on the substrate 210 that exceed the internal cohesive strength and/or tensile strength of the substrate 210.

For example, if the substrate 210 includes a resin-bonded nonwoven portion, the temperature of the transfer roll 230 and/or molten thermoplastic composition may rise above the melting temperature of the resin, thereby potentially degrading the internal cohesive strength and/or tensile strength of the substrate 210. Alternatively, a nonwoven substrate may include fibers that have a melting temperature similar to the temperature of the transfer roll 230 and/or molten thermoplastic composition, thereby potentially degrading the internal cohesive strength and/or tensile strength of the substrate 210.

In either instance, the roll temperatures and/or molten thermoplastic composition temperature may need to be controlled to maintain the integrity of the substrate while transferring the molten thermoplastic composition. For example, the backup roll 220 may be cooled to, in turn, cool the substrate 210 to maintain its internal cohesive strength.

In another alternative, heating of the transfer roll 230 and/or backup roll 220 may be used to enhance the internal cohesive strength and/or tensile strength of the substrate 210. For example, if the substrate 210 includes multicomponent fibers or fibers having different compositions, some consolidation of the fibers or other components in the substrate 210 may be caused by heating the substrate 210 while transferring the molten thermoplastic composition from the transfer roll 230 to the substrate 210. That consolidation may improve the integrity of the substrate by forming a skin layer or other strength-enhancing structure on or within the substrate 210. Some exemplary processes may be described in, e.g., U.S. Patent No. 5,470,424 (Isaac et al.).

Having thus described some of the basic characteristics of composite webs and methods and systems of manufacturing them according to the present invention, a specific application of the present invention will now be described.

In that regard, FIG. 5 depicts one example of a disposable diaper 470 that may include one or more components manufactured according to the present invention. The diaper 470 includes a body 472 that may be manufactured of various materials useful in connection with diapers. Some exemplary diaper constructions may be described in, e.g., U.S. Patent Nos. 5,399,219 (Roessler et al.) and 5,685,873 (Bruemmer et al.).

The diaper 470 includes fastening tabs 474 that extend laterally from the body 472 and are connected to opposing lateral ends of at least one waistband portion 473 for securing the waistband sections of the article about a wearer during the use of the article. The fastening tabs 474 are preferably formed of composite webs according to the principles of the present invention

The diaper 470 also includes fastening tab receiving areas 476 that are located in a waistband portion 475 at the opposite end of the diaper 470. Fastening tabs 474 may be attached to the fastening tab receiving areas 476 to retain the diaper on a wearer. Although two receiving areas are depicted in FIG. 5, it will be understood that in some instances a single larger receiving area may be provided that extends substantially across the diaper in the area of waistband 475.

Fastening tab receiving area 476 can have any suitable construction to retain the fastening tab 474. For example, if the fastening tab 474 includes hooks formed thereon, the receiving area 476 may be constructed of, e.g., loop material that cooperates with the hooks to retain the fastening tab 474 on the receiving area 476.

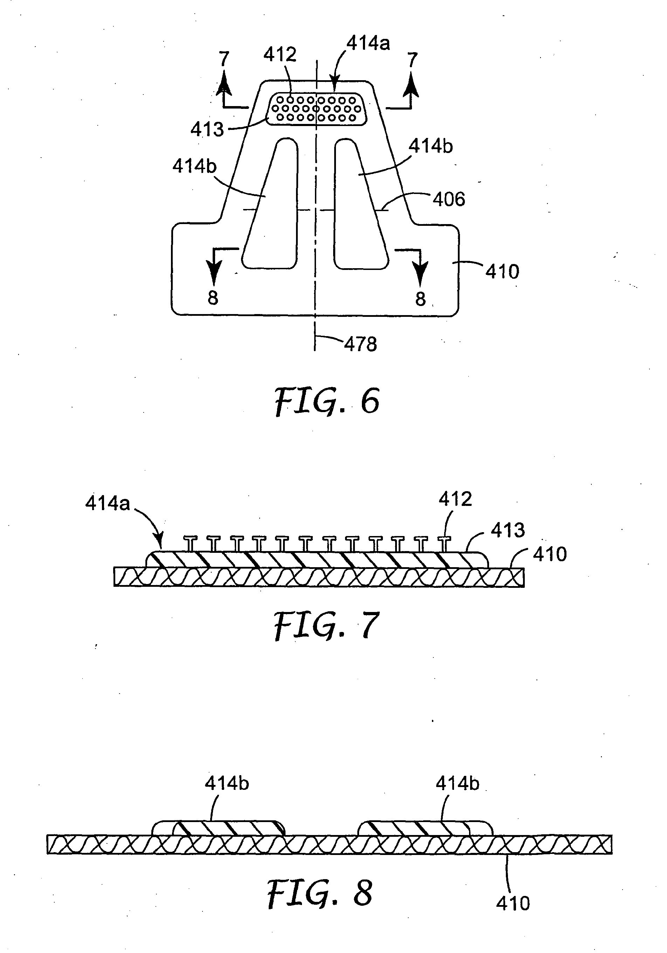

FIGS. 6-8 depict various views of one of the fastening tabs 474 attached to diaper 470 to illustrate various features of the present invention. Fastening tab 474 includes a substrate 410 on which a variety of different discrete polymeric regions are located. The different discrete polymeric regions provide a mechanical fastener (414a) for attaching the tab 474 to a complementary surface (e.g., receiving surface 476 in FIG. 5) and elastic elements (414b) to provide elasticity to the fastening tab 474. The tab 474 preferably includes an elongation axis 478 seen in FIG. 6.

Discrete polymeric region 414a is provided proximate the distal end of the tab 474. FIG. 7 is a cross-sectional view taken along line 7-7 in FIG. 6 and depicts structures 412 protruding from a base 413 of the discrete polymeric region 414a. In the embodiment depicted in FIG. 7, the structures 412 are fastening structures in the form of a plurality of capped stems, although many other suitable fastening structures could be used in place of capped stems.

The depicted stems 412 are oriented substantially perpendicular to the base 413 of the discrete polymeric region 414a, as well as the underlying substrate 410, although it will be understood that the exact form and structure of the stems 412 may vary based on the intended use of the composite web. Furthermore, although all of the stems 412 are shown as having the same size and shape, it will be understood that a variety of differently sized and/or shaped stems may be provided as desired based on the intended use of the fastening tab 474.

The discrete polymeric region 414a may be formed of elastomeric or nonelastomeric materials, although it may be preferred that the discrete polymeric region 414a be manufactured of nonelastomeric materials if it is desired that the discrete polymeric region 414a also function to distribute stresses over the width of the fastening tab 474 (where the width is measured generally transverse to the elongation axis 478 depicted in FIG. 6). It may be desirable to distribute the forces applied during elongation of the tab 474 to reduce or prevent necking or roping of the tab 474. Force distribution may also be helpful to improve uniformity in the forces seen across the width of the tab 474.

The fastening tab 474 also includes discrete polymeric regions 414b that preferably function as elastic elements to provide elasticity to the tab 474 if the substrate 410 is nonelastic. If the substrate 410 is elastic, the discrete polymeric regions 414b may still function as elastic elements that enhance the elasticity of the tab 474. To function as elastic elements, the discrete polymeric regions 414b are formed of an elastomeric thermoplastic composition as defined above.

Although the substrate 410 is preferably extensible, a nonextensible substrate 410 can be made extensible by, e.g., providing slits 406 in the substrate 410. The slits 406 are preferably spanned by at least one of the discrete elastomeric polymeric regions 414b. Some exemplary slitting processes to provide or improve extensibility of a substrate are described in International Publication No. WO 96/10481 (Abuto et al.). Other techniques may also be used to provide or improve the extensibility of substrates used in connection with the present invention. For example, the mechanical stretching processes described in U.S. Patent Nos. 4,223,059 (Schwarz) and 5,167,897 (Weber et al.) may be used to provide or improve extensibility.

In the depicted embodiment, the discrete polymeric regions 414b are located on the same surface of the substrate 410 as the discrete polymeric region 414a. Each of the discrete polymeric regions 414b preferably includes a length that is substantially aligned with the elongation axis 478. For the purposes of the present invention, the length of the discrete polymeric regions 414b is the longest straight line dimension of the discrete polymeric regions 414b as measured along the surface of the substrate 410.

Another feature of the discrete polymeric regions 414b is their nonuniform or changing width. As seen in FIG. 6, the discrete polymeric regions 414b become wider when moving away from the discrete polymeric region 414a. If the height or thickness of the discrete polymeric regions 414b above the surface of the substrate 410 is constant, the net result of the changing width depicted in FIG. 6 is that the amount of elastomeric material in the discrete polymeric regions 414b increases when moving away from the discrete polymeric region 414a. The changing bulk of elastomeric material may, e.g., provide a tab 474 that has different elasticity and/or elongation properties at different locations along the elongation axis 478. Many other variations in the distribution of elastomeric material in the discrete polymeric regions 414b may be used to tailor the elasticity and/or elongation properties of the fastening tab 474, e.g., adjusting the thickness of the polymeric regions, the materials used, etc.

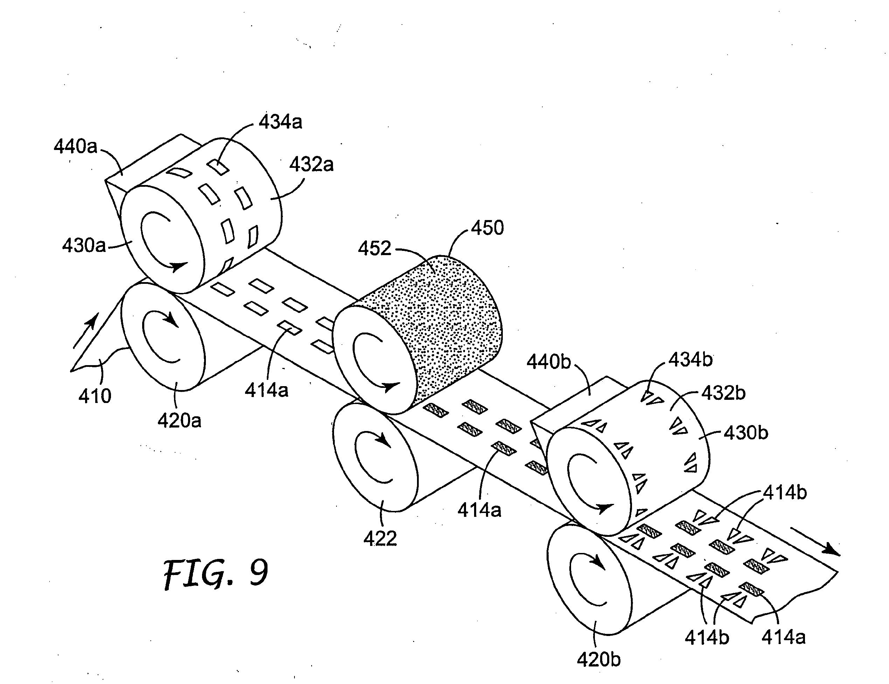

FIG. 9 depicts one system that may be used to manufacture, e.g., the fastening tabs 474 of FIGS. 6-8 where all of the discrete polymeric regions are located on the same surface of the substrate 410. The system includes a substrate 410 that moves through the system as indicated by the arrows at the left and right ends of the web path, as well as by the rotation arrows provided on the various rolls.

The substrate 410 is first directed into a first transfer nip formed by backup roll 420a and first transfer roll 430a. First transfer roll 430a includes depressions 434a formed in its exterior surface 432a. A molten thermoplastic composition delivery apparatus 440a is located on transfer roll 430a to fill the depressions 434a with the desired molten thermoplastic composition.

After passing through the first transfer nip, substrate 410 includes discrete polymeric regions 414a located thereon. Because the discrete polymeric regions 414a on the fastening tab 474 preferably include some structure formed thereon to provide a fastening mechanism, the substrate 410 including discrete polymeric regions 414a may be directed into a forming nip provided by a forming tool 450 and backup roll 422. The forming nip is downstream of the transfer nip in the depicted system.

Although the forming tool 450 is depicted as providing the forming nip using backup roll 422, it should be understood that, alternatively, the transfer nip and the forming nip could be formed using the same backup roll. Using the same backup roll for both the transfer nip and the forming nip, may, e.g., be beneficial in that fewer system components and/or floorspace may be required for the system.

In systems and methods where the transfer nip and the forming nip are formed using different backup rolls, the thermoplastic composition in the discrete polymeric regions 414a may no longer be sufficiently molten to form structures in the forming nip. If this is the case, the discrete polymeric regions 414a on the substrate 410 may need to be heated before passing through the forming nip (by, e.g., contact or noncontact heat sources).

The forming tool 450 is provided in the form of a roll and includes cavities 452 formed in its surface. Forming tools such as that depicted in FIG. 9 are well known to those of skill in the art. Some forming tools are described in, for example, U.S. Patent Nos. 4,984,339 (Provost et al.), 5,077,870 (Melbye et al.), 5,755,015 (Akeno et al.), 5,868,987 (Kampfer et al.), 6,132,660 (Kampfer), 6,190,594 B1 (Gorman et al.), 6,287,665 B1 (Hammer), etc.

The forming tool 450 and/or backup roll 422 may be heated or cooled to a selected temperature based on the properties of the thermoplastic composition being formed to enhance forming of the discrete polymeric regions by the cavities 452 in the forming tool 450. For example, it may be desirable to heat or cool the forming tool 450 to enhance the forming process. Depending on the speed of the process and other factors, the discrete regions of thermoplastic composition located on substrate 410 may also advantageously retain some of their molten nature as transferred to the substrate 410.

In any event, a portion of the thermoplastic composition in discrete polymeric regions 414a located on the substrate 410 enters the cavities 452 on the forming tool 450. As a result, structures such as the stems depicted in FIG. 9 (see FIGS 6 and 7 also) may be formed in the discrete polymeric regions 414a located on substrate 410.

In some instances, the thermoplastic composition provided in discrete regions on the substrate 410 may possess properties (e.g., viscosity, etc.) such that the thermoplastic composition replicates the shape of the cavities 452 provided in the forming tool 450. As used herein, the term "replicates" (and variations thereof) includes complete replication as well as partial replication of the shape of the cavities 452 by the thermoplastic composition. In other instances, the properties (e.g., viscosity, etc.) may result in forming of the thermoplastic composition on the substrate 410 into shapes that, although they differ from the shape of the thermoplastic composition before forming by the forming tool 450, do not replicate the shape of the cavities 452 as described above.

Following transfer and forming of the discrete polymeric regions 414a, the substrate 410 is directed into a second transfer nip at which the discrete polymeric regions 414b are deposited on the substrate 410. The second transfer nip includes a second transfer roll 430b and a backup roll 420b, as well as a molten thermoplastic composition delivery apparatus 440b located on transfer roll 430b to fill the depressions 434b formed in exterior surface 432b of transfer roll 430b with the desired molten thermoplastic composition.

As the substrate 410 exits the second transfer nip, it includes a second set of discrete polymeric regions 414b in addition to discrete polymeric regions 414a, with both sets being located on the same surface of the substrate 410. The different sets of discrete polymeric regions 414a and 414b may be manufactured of the same or different thermoplastic compositions.

Because the substrate 410 includes the set of discrete polymeric regions 414a as delivered to the second transfer nip, it may be desirable if, e.g., the backup roll structures discussed in connection with FIGS. 4B and 4C be used to provide additional force that may assist in the transfer process.

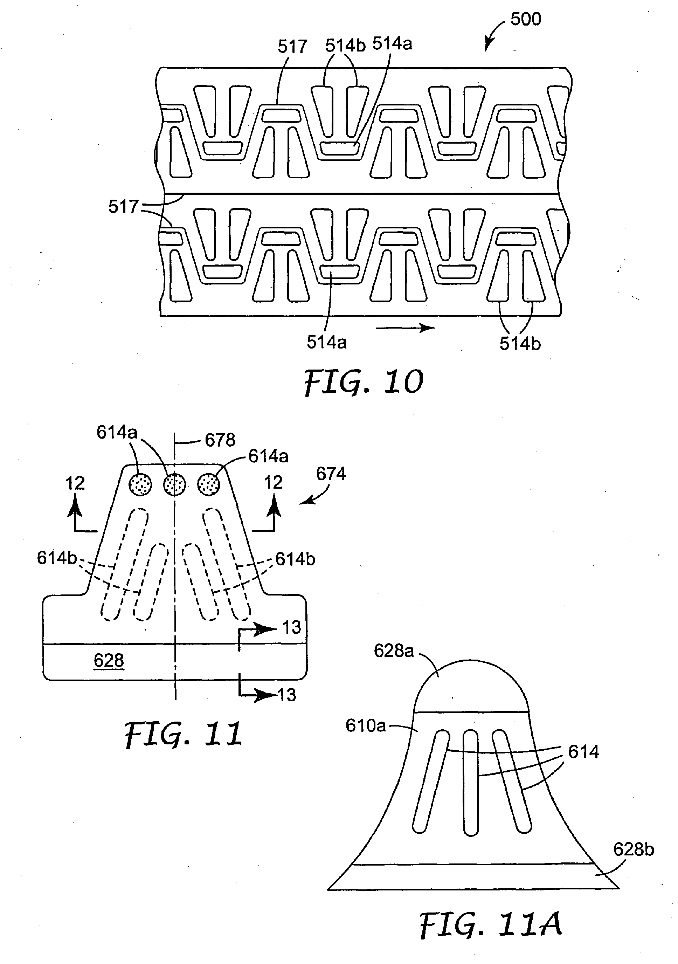

FIG. 10 depicts one composite web 500 that may be, at least in part, manufactured using the system of FIG. 9. The composite web 500 includes a variety of different discrete polymeric regions 514a and 514b located thereon. In addition, the composite web 500 includes lines of separation 517 that define the boundaries of a number of different fastening tabs similar to those described above with respect to FIGS 6-8. The lines of separation 517 define a nested configuration of fastening tabs including the discrete polymeric regions 514a and 514b in a manner that may reduce waste when the composite web 500 is separated along the lines of separation 517 to provide the desired fastening tabs. The lines of separation 517 may take on any suitable form that facilitates separation of the composite web 500 along the lines of separation, e.g., score lines, lines of weakness, lines of perforations, etc.

The composite web 500 preferably has a length that extends along the direction of the straight line of separation 517 extending from left to right in FIG. 10. Although the composite web 500 includes only two pairs of nested tabs across the width of the composite web 500 (where width is transverse to length), it will be understood that any desired number of nested pairs of tabs may be provided in a single composite web according to the present invention.

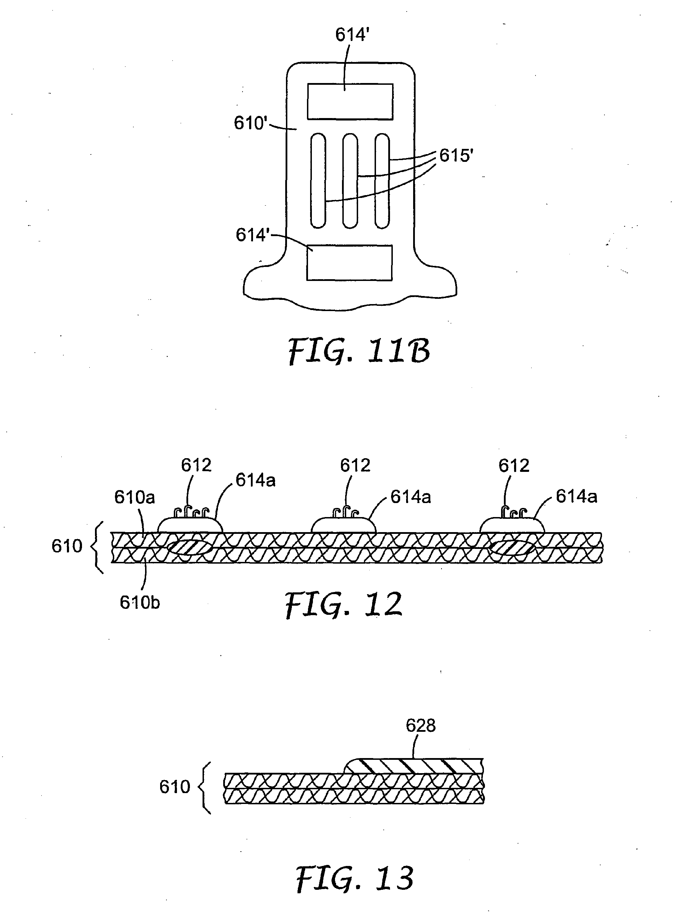

FIGS. 11-13 depict various views of another fastening tab 674 that may be used in connection with a garment, e.g., a diaper. Fastening tab 674 includes a laminated substrate 610 on and in which a variety of different discrete polymeric regions are located. The different discrete polymeric regions provide a mechanical fastener (using discrete polymeric regions 614a) for attaching the tab 674 to a complementary surface and elastic elements (614b) to provide elasticity to the fastening tab 674. The tab 674 preferably includes an elongation axis 678 seen in FIG. 11.

Mechanical fasteners in the form of discrete polymeric regions 614a are provided proximate the distal end of the tab 674. FIG. 12 is a cross-sectional view taken along line 12-12 in FIG. 11 and depicts structures 612 (e.g., hooks) protruding from the discrete polymeric regions 614a. In the embodiment depicted in FIG. 12, the structures 612 are in the form of hooks, although many other suitable structures could be used in place of the depicted hooks. The discrete polymeric regions 614a used to provide mechanical fasteners to the tab 674 may be formed of elastomeric or nonelastomeric materials.

The fastening tab 674 also includes discrete polymeric regions 614b that preferably function as elastic elements to provide elasticity to the tab 674 if the substrate 610 is nonelastic. If the substrate 610 is elastic, the discrete polymeric regions 614b may still function as elastic elements that enhance the elasticity of the tab 674. To function as elastic elements, the discrete polymeric regions 614b are formed of an elastomeric thermoplastic composition as defined above.

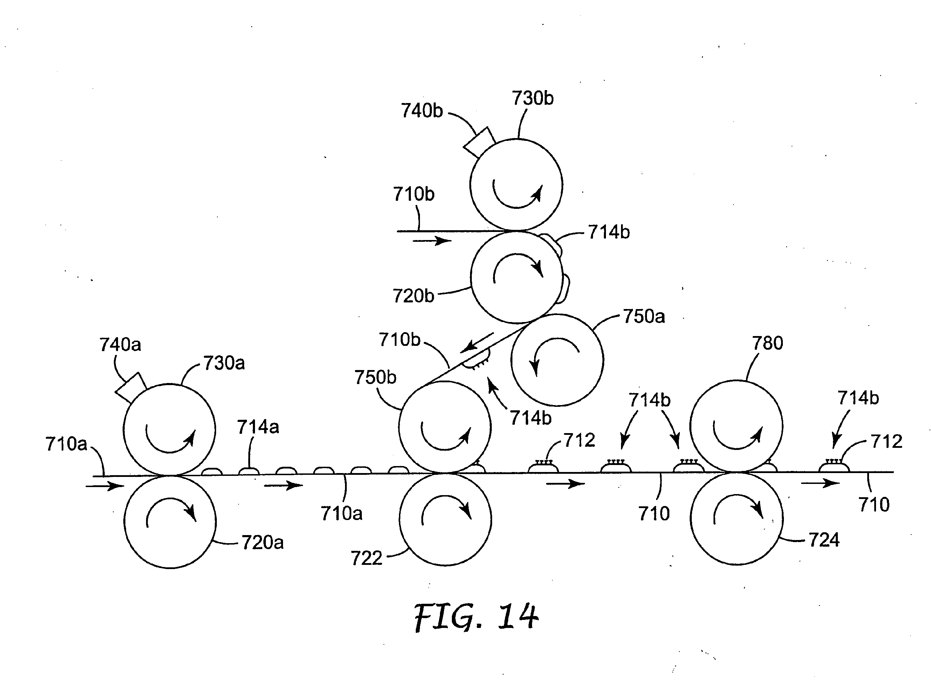

In the depicted embodiment, the discrete polymeric regions 614b are located between substrates 610a and 610b of laminated substrate 610. This construction may be desirable to protect the elastomeric discrete polymeric regions 614b and to provide a softer feel to the tab 674. One method and system of manufacturing a laminated composite web is described below in connection with FIG. 14.

Each of the discrete polymeric regions 614b preferably includes a length that is substantially aligned with the elongation axis 678. For the purposes of the present invention, the length of the discrete polymeric regions 614b is the longest straight line dimension of the discrete polymeric regions 614b as measured along the surface of the substrate 610.

Unlike the polymeric regions with a variable width as depicted n FIG. 6, the polymeric regions 614b have a generally consistent width over their length. Variable elasticity and/or elongation may, however, be obtained by providing more discrete polymeric regions with different lengths, such that their combined bulk or mass becomes larger when moving away from the discrete polymeric regions 614a along the elongation axis 678. If the height or thickness of the discrete polymeric regions 614b measured through the thickness of the substrate 610 is constant, the net result of the arrangement depicted in FIG. 11 is that the amount of elastomeric material in the discrete polymeric regions 614b increases when moving away from the discrete polymeric regions 614a. The varying bulk of elastomeric material may, e.g., provide a tab 674 that has varying elasticity and/or elongation properties when moving along the elongation axis 678. Many other variations in the distribution of elastomeric material in the discrete polymeric regions 614b may be used to tailor the elasticity and/or elongation properties of the fastening tab 674,e.g., varying the thickness, materials, etc.

FIGS. 11 and 13 also depict another optional feature in the form of a bonding site 628 provided on the substrate 610. The bonding site 628 may be provided to assist in the attachment of fastening tab 674 to a larger article, e.g., a diaper, gown, etc. To assist in attachment, the bonding site 628 may take a variety of configurations. For example, the bonding site may be a consolidated area of a nonwoven or woven fabric amenable to thermal or other consolidation techniques. Alternatively, or in addition to consolidation, the bonding site may include one or more materials that assist in bonding, e.g., block copolymers, ethylene vinyl acetates, tackified ethylene vinyl acetates, adhesives (pressure sensitive, curable, heat activated, etc.), amorphous polyolefins, etc. The specific selection of materials to locate in the bonding site 628 will depend on the type of bonding to be performed and the materials to be bonded.

One advantage of the bonding site 628 is that it can be formed of materials that are particularly amenable to the attachment technique to be used, e.g., heat sealing, ultrasonic welding, etc. Another advantage is that the bonding site can be sized such that it is large enough to accomplish its function, but not so large that any materials used in the bonding site are wasted. Depending on the composition of the materials to be provided at the bonding site, it may be formed by the transfer methods described herein if a thermoplastic composition is to be used in the bonding site 628.

In some disposable articles, e.g., training pants, bonding sites may be provided to bond an element to a like element, where a bonding site is located on one or both of the elements. FIG. 11A depicts an article that includes two bonding sites 628a and 628b located on opposing sides of an area that includes discrete elastomeric polymeric regions 614 located on a substrate 610. If the article depicted in FIG. 11A is to be used as, e.g., a fastening tab, it may be preferred that one or both of the bonding sites 628a and 628b be adapted to receive a mechanical fastener that may be bonded to the tab separately. Alternatively, an adhesive (e.g., pressure sensitive, curable, heat activated, etc.) or cohesive material could be provided within one or both of the bonding sites 628a and 628b.

FIG. 11B depicts another alternative article including discrete polymeric regions on a substrate in accordance with the present invention. The article is formed on a substrate 610' and includes two discrete polymeric regions 614' that may include, e.g., hooks, stems, capped stems, or other fastening structures. At least one, and preferably more than one, discrete elastomeric polymeric regions 615' are located between the two discrete polymeric regions 614' on the article.

FIG. 14 depicts one system that may be used to manufacture, e.g., the fastening tabs 674 of FIGS. 11-13 where some discrete polymeric regions are located on the exterior surface of the substrate 610 and others are located between substrates forming the laminated substrate 610. The system includes a web path that moves through the system as indicated by the arrows at the left and right ends of the web path, as well as by the rotation arrows provided on the various rolls.

The substrate 710a is directed into a first transfer nip formed by backup roll 720a and first transfer roll 730a. First transfer roll 730a includes depressions formed in its exterior surface. A molten thermoplastic composition delivery apparatus 740a is located on transfer roll 730a to fill the depressions with the desired molten thermoplastic composition. After passing through the first transfer nip, substrate 710a includes discrete polymeric regions 714a located thereon.

The system also includes a second substrate 710b that is directed into a second transfer nip formed by backup roll 720b and second transfer roll 730b that includes depressions formed in its exterior surface. A molten thermoplastic composition delivery apparatus 740b is located on transfer roll 730b to fill the depressions with the desired molten thermoplastic composition. After passing through the second transfer nip, substrate 710b includes discrete polymeric regions 714b located thereon.

Because the discrete polymeric regions 714b preferably include some structure formed thereon to provide a fastening mechanism, the substrate 710b including discrete polymeric regions 714b may be directed into a forming nip provided by a forming tool 750a and backup roll 720b. The forming nip is downstream of the transfer nip in the web path of substrate 710b.

The forming tool 750a is provided in the form of a roll and includes cavities formed in its surface. Forming tools such as that depicted in FIG. 11 are well known to those of skill in the art. Some forming tools are described in, for example, U.S. Patent Nos. 4,984,339 (Provost et al.), 5,077,870 (Melbye et al.), 5,755,015 (Akeno et al.), 5,868,987 (Kampfer et al.), 6,132,660 (Kampfer), 6,190,594 B1 (Gorman et al.), 6,287,665 B1 (Hammer), etc.

The forming tool 750a and/or backup roll 720b may be heated or cooled to a selected temperature based on the properties of the thermoplastic composition being formed to enhance forming of the discrete polymeric regions by the cavities in the forming tool 750a. For example, it may be desirable to heat or cool the forming tool 750a to enhance the forming process. Depending on the speed of the process and other factors, the discrete regions of thermoplastic composition located on substrate 710b may also advantageously retain some of their molten nature as transferred to the substrate 710b.

In any event, a portion of the thermoplastic composition in discrete polymeric regions 714b located on the substrate 710b enters the cavities on the forming tool 750a. As a result, structures such as the stems depicted in FIG. 11 may be formed in the discrete polymeric regions 714b located on substrate 710b.

Following transfer and forming of the discrete polymeric regions 714b, the substrates 710a and 710b are directed into a lamination nip formed by rolls 750b and 722, where the substrates are laminated such that the discrete polymeric regions 714a are located between substrates 710a and 710b and the discrete polymeric regions 714b are located on a surface of the laminated substrate 710.

The lamination nip formed by rolls 722 and 750b may cause a portion of the thermoplastic composition in the discrete polymeric regions 714a to infiltrate the substrate 710b (and/or encapsulate at least a portion of at least some fibers, if any, present in the substrate 710b). If that mechanism is used to accomplish lamination of the substrates, no additional materials or processes need be formed to complete the lamination.

Lamination in the absence of any other agents or techniques, may need to occur while the polymer regions 714a are still in a somewhat molten state such that they can bond with counterpart discrete polymeric regions on the opposing substrate or to the opposing substrate itself. The lamination between substrates 710a and 710b may alternatively be assisted by a variety of materials and/or techniques known to those skilled in the art, e.g., thermal bonding, adhesives, resins, tie films/webs, etc. See, e.g., U.S. Patent Nos. 2,787,244 (Hickin); 3,694,867 (Stumpf); 4,906,492 (Groshens); 5,685,758 (Paul et al.); and 6,093,665 (Sayovitz et al.).

The laminated constructions described in connection with FIGS. 11-14 may be useful, for example, to provide a cloth-like or softer feel or appearance, breathability, porosity, etc. on both sides of the composite web. This is in contrast to the composite webs in which all of the discrete polymeric regions are located on an exposed surface of the composite web. A laminated composite web structure such as that seen in, e.g., FIGS. 11 and 12 may also be used to provide different properties on opposite sides of the composite web structure. For example, the porosity or other properties may differ between the different substrates 710a and 710b.

The lamination nip formed by rolls 750b and 722 may also function as a deforming station to deform the structures formed on discrete polymeric regions 714b if so desired. The deforming station may, for example, perform a variety of processes to deform the structures on discrete polymeric regions 714b after they are formed at the forming nip. Examples of some suitable processes that may be performed at the deforming station include, but are not limited to, trimming, shaving, abrading heating or melting (using a contact or noncontact heat source), bending or otherwise distorting the structures. Where the structures are stems, the deforming may include, e.g., forming a cap on the stem, forming a hook on a stem, bending the stem, etc. Some potential apparatus and processes are described in, for example, U.S. Patent Nos. 5,077,870 (Melbye et al.), 5,868,987 (Kampfer et al.), 6,039,911 (Miller et al.), 6,054,091 (Miller et al.), and 6,132,660 (Kampfer).

After the laminated substrate 710 exits the lamination nip, it may be directed into an optional station formed by rolls 780 and 724. This station may also function as a deforming station in addition to lamination nip or in place of the lamination nip. Another potential process that may be performed by rolls 780 and 724 is the formation of lines of separation in the laminated substrate 710 similar to lines of separation 517 discussed in connection with FIG. 10 above.

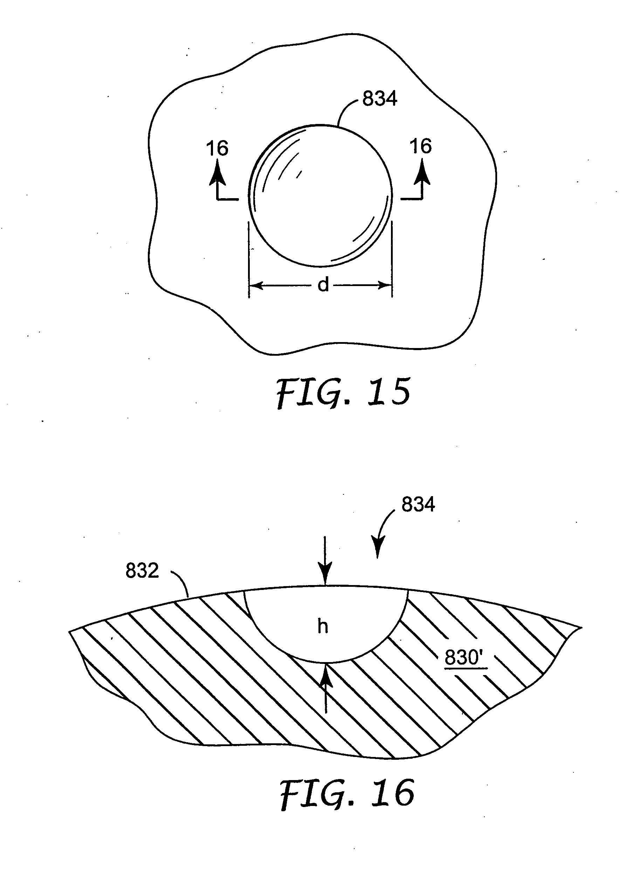

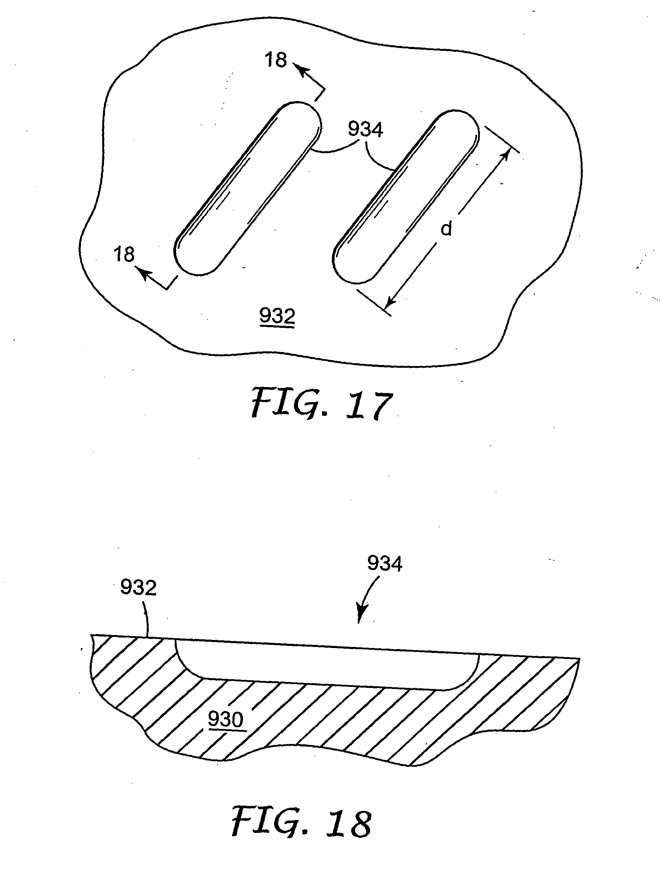

FIG. 15 is a plan view of one exemplary depression 834 in transfer roll 830 of the present invention, while FIG. 16 is a cross-sectional view of the depression 834 taken along line 16-16 in FIG. 15. The depression 834 has a circular footprint (i.e. shape of the opening into the depression 834 at the surface 832 of the roll) with a diameter represented by the letter *d*. The depression 834 has a depth (represented by the letter *h*) measured from the exterior surface 832 of the transfer roll 830.

Transfer rolls used in connection with the present invention may preferably include depressions that are large enough to form discrete polymeric regions of sufficient size to support, for example, the formation of multiple stems or other structures in each of the discrete polymeric regions. The depressions may be characterized in a variety of manners. For example, the depressions 834 may be characterized in terms of the area occupied by their footprint on the exterior surface of the forming tool, a maximum dimension of the footprint (in any direction on the surface of the roll), the volume of the depression, the shape of the footprint, etc.

When characterized in terms of the area occupied by the footprint of the depressions, each of the depressions 834 may have a footprint with an area of about 4 square millimeters (mm²) or more. In other situations, each of the depressions 834 may have footprints with an area of about 8 mm² or more.

Another manner in which the depressions may be characterized is in terms of the largest footprint dimension as measured on the surface 832 of the transfer roll 830. For a depression with a circular footprint as seen in FIGS. 15 and 16, the largest dimension is the same in all directions, but the depressions used in connection with the present invention may take any desired shape (e.g. elongated, irregular, etc.) in which the largest dimension will occur in one or more directions on the exterior surface of the transfer roll 830, but not in others. When characterized in terms of the largest footprint dimension, it may be that the depressions have a largest footprint dimension of about 2 mm or more, in some instances about 5 mm or more.

Yet another manner in which the depressions used in connection with the present invention may be characterized is in terms of volume. For example, the depressions may have a depression volume of at least about three (3) cubic millimeters (mm³) or more, or alternatively, a depression volume of about five (5) cubic millimeters. Volume of the discrete polymeric regions may be important to provide enough of the thermoplastic composition to adequately enter the cavities in a forming tool. Depression volume may also be important because at least some of the molten thermoplastic composition may be retained within the depression during the transfer process, i.e., the depression volume may preferably be oversized relative to the preferred volume of the discrete polymeric regions to compensate for retention of thermoplastic composition within the depressions.

FIG. 17 depicts two depressions 934 formed in an exterior surface 932 of a transfer roll, with FIG. 18 being a cross-sectional view of one of the depressions 934 taken along line 18-18 in FIG. 17. The depressions 934 have elongated shapes in the form of, e.g., a trough. When compared to the circular depression 834 seen in FIGS. 15 and 16, the longer depressions 934 of FIGS. 17 and 18 would have a larger footprint dimension along their elongated direction than transverse to their elongated direction.

The orientation of the depressions 934 may be selected based on a variety of factors. The elongated depressions 934 may be aligned in the machine direction (i.e., the direction of travel of a substrate), in the cross-web direction (i.e., transverse to the direction of travel of the substrate), or any other orientation between machine direction or cross-web direction.

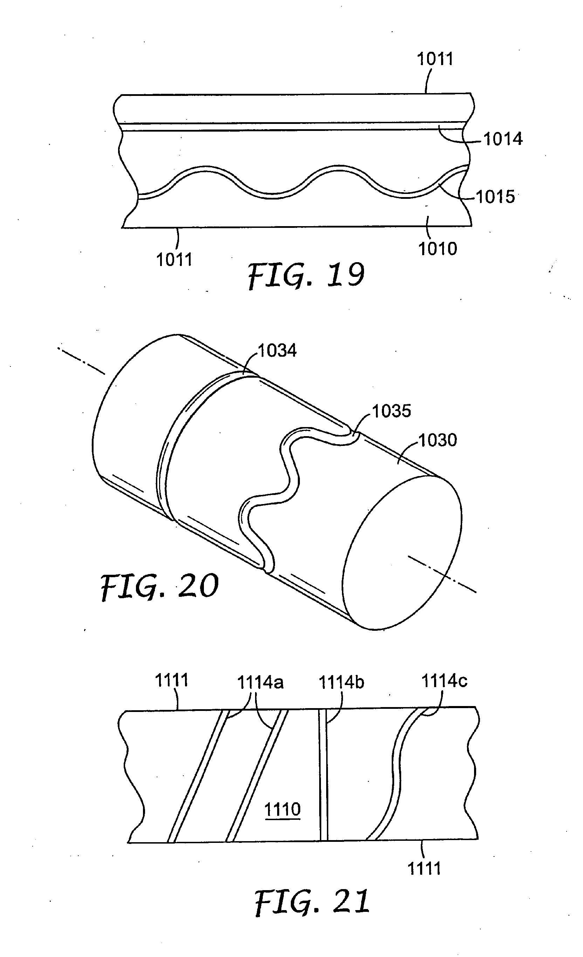

FIGS. 19 & 20 depict another variation associated with the methods of manufacturing composite webs according to the present invention. FIG. 19 depicts, in a plan view, a portion of a composite web manufactured according to the present invention. The composite web includes a substrate 1010 on which two discrete polymeric regions 1014 and 1015 are located. The substrate includes two opposing edges 1011 that extend over the length of the composite web and, together, define the longitudinal length of the composite web.

Discrete polymeric region 1014 is provided in the shape of a line of the thermoplastic composition material deposited on the substrate 1010 along the general direction of the longitudinal length of the composite web. The discrete polymeric region 1014 may be continuous along the longitudinal length of the composite web as shown in FIG. 19.

Discrete polymeric region 1015 is a variation of discrete polymeric region 1014 in that it is provided in an undulating shape as compared to the relative straight linear shape of the discrete polymeric region 1014. The undulating shape of the discrete polymeric region 1015 also, however, extends along the direction of the longitudinal length of the composite web. Further, the discrete polymeric region 1015 may be continuous along the longitudinal length of the composite web as shown in FIG. 19.

FIG. 20 is a perspective view of one transfer roll 1030 that may be used to transfer thermoplastic compositions in the shapes seen in FIG. 19 according to the methods of the present invention. The transfer roll 1030 includes a depression 1034 that preferably extends continuously around the outer circumference of the roll 1030 to form the discrete polymeric region 1014 as depicted in FIG. 19. The transfer roll 1030 also includes a depression 1035 that also extends around the outer circumference of the roll 1030 to form the discrete polymeric region 1015 as depicted in FIG. 19.

FIG. 21 depicts another variation associated with the methods of manufacturing composite webs according to the present invention. FIG. 21 depicts, in a plan view, a portion of a composite web manufactured according to the present invention. The composite web includes a substrate 1110 on which discrete polymeric regions 1114a, 1114b, and 1114c are located, with the discrete polymeric regions extending across the width of the substrate. The substrate 1110 includes two opposing edges 1111 that extend over the length of the composite web and, together, define the width and the longitudinal length of the composite web.

Each of the discrete polymeric regions 1114a, 1114b, and 1114c is provided in the shape of a line of the thermoplastic composition material deposited on the substrate 1110 in a generally cross-web direction, i.e., extending between the opposing edges 1111 of the substrate 1110. The discrete polymeric regions 1114a, 1114b, and 1114c present variations from straight lines 1114a and 1114b to undulating line 1114c. Many other variations in placement, shape and/or orientation of discrete polymeric regions may be envisioned in connection with methods according to the present invention.

In addition to the deposition of thermoplastic polymer in discrete regions, it is also contemplated that additional materials can be coated onto a major surface of the substrate using known methods. Such materials could be, for example adhesives, as described in, e.g., U.S. Patent Nos. 5,019,071 (Bany et al.); 5,028,646 (Miller et al.); and 5,300,057 (Miller et al.); or cohesives as described in, e.g. U.S. Patent Nos. 5,389,438 (Miller et al.) and 6,261,278 (Chen et al.).

### EXAMPLES

The following examples are provided to enhance understanding of the present invention. They are not intended to limit the scope of the invention.

### Example 1

A web of the present invention was produced using apparatus similar to that shown in Fig. 8. A 51 mm diameter single screw extruder was used to deliver a molten polymer consisting of ultra low density polyethylene (ENGAGE 8400, DupontDow Elastomers) at a melt temperature of approximately 207°C to a neck tube. The neck tube was positioned such that a thick strand of molten polymer was extruded vertically downward onto the exterior surface 32 of an oil-heated steel transfer roll 30 having a diameter of 23 cm. The exterior surface of the transfer roll was machined using a computer controlled milling machine to have depressions in the shape of grooves parallel to the roll axis 25.4 cm long, 2.3 mm in width, 1.3 mm in depth, arranged with a center-to-center spacing between grooves of 1.0 cm. After the depressions were filled or partially filled with the molten polymer, any excess molten polymer was removed from the exterior surface of the transfer roll by a brass doctor blade 42 having a thickness of 1.5 mm at the contact point with the roll, acting against and normal to the exterior surface of the transfer roll. The excess molten polymer formed a small rolling bank of polymer contained in a trough formed by the doctor blade and two side walls pressed firmly against the transfer roll using a pressure of 88 N/lineal cm. The transfer roll was at approximately 204°C. After the wiping action of the doctor blade, the transfer roll continued to rotate until the depressions and the molten polymer they contain were forced into contact with a nonwoven substrate (HEF-140-070 spunlaced polyester, 30 grams/m², BBA Nonwovens) against a rubber backup roll 20 (66°C) using a nip pressure of 88 N/lineal cm. Transfer of some of the molten polymer from the depressions to the nonwoven substrate occurred. A portion of the molten polymer in the depressions remained in the depressions while the substrate pulled away from the transfer roll. As a result, the molten polymer tended to elongate or string between the depressions in the transfer roll and the substrate. A hot wire 44 was used to sever any strands of molten polymer formed as the substrate separated from the transfer roll. The basis weight of each transferred molten polymer region was 347 grams/m². The cumulative basis weight of the transferred polymer regions on the nonwoven substrate was 47 grams/m².

### Example 2

A web was produced as in Example 1 except an SEBS block copolymer elastomer (KRATON G-1657, Shell Chemical) was used as the molten polymer. The temperature of the molten polymer was approximately 249°C and the transfer roll was at approximately 246°C. A nip pressure of 53 N/lineal cm was used. The basis weight of each transferred molten polymer region was 529 grams/m². The cumulative basis weight of the transferred polymer regions on the nonwoven substrate was 72 grams/m².

### Example 3

A web was produced as in Example 1 except the temperature of the molten polymer was approximately 223°C and the transfer roll was at approximately 218°C. The temperature of the backup roll was approximately 38°C. A nip pressure of 88 N/lineal cm was used. The basis weight of each transferred molten polymer region was 449 grams/m². The cumulative basis weight of the transferred polymer regions on the nonwoven substrate was 61 grams/m².

### Example 4

A web was produced as in Example 1 except a blend of ENGAGE 8400 polyethylene - 50% and ENGAGE 8100 polyethylene - 50%, was used as the molten polymer. The temperature of the molten polymer was approximately 218°C and the transfer roll was at approximately 218°C. The temperature of the backup roll was approximately 38°C. A nip pressure of 88 N/lineal cm was used. The basis weight of each transferred molten polymer region was 321 grams/m². The cumulative basis weight of the transferred polymer regions on the nonwoven substrate was 44 grams/m².

### Example 5

A web was produced as in Example 1 except a blend of ENGAGE 8400 polyethylene - 75% and ENGAGE 8100 polyethylene - 25%, was used as the molten polymer. The temperature of the molten polymer was approximately 223°C and the transfer roll was at approximately 218°C. The temperature of the backup roll was approximately 38°C. A nip pressure of 88 N/lineal cm was used. The basis weight of each transferred molten polymer region was 491 grams/m². The cumulative basis weight of the transferred polymer regions on the nonwoven substrate was 67 grams/m².

### Example 6

A web was produced as in Example 2 except the temperature of the molten polymer was approximately 251°C and the transfer roll was at approximately 246°C. The temperature of the backup roll was approximately 38°C. A nip pressure of 88 N/lineal cm was used. The basis weight of each transferred molten polymer region was 656 grams/m². The cumulative basis weight of the transferred polymer regions on the nonwoven substrate was 90 grams/m².

### Example 7

A web was produced as in Example 1 except ENGAGE 8200 polyethylene was used as the molten polymer. The temperature of the molten polymer was approximately 204°C and the transfer roll was at approximately 204°C. The temperature of the backup roll was approximately 38°C. A nip pressure of 175 N/lineal cm was used. The basis weight of each transferred molten polymer region was 767 grams/m². The cumulative basis weight of the transferred polymer regions on the nonwoven substrate was 104 grams/m².

### Example 8

A web was produced as in Example 1 except an elastomeric polyurethane (58-680, Noveon) was used as the molten polymer. The temperature of the molten polymer was approximately 210°C and the transfer roll was at approximately 210°C. The temperature of the backup roll was approximately 38°C. A nip pressure of 175 N/lineal cm was used. The basis weight of each transferred molten polymer region was 495 grams/m². The cumulative basis weight of the transferred polymer regions on the nonwoven substrate was 68 grams/m².

### Example 9

A web was produced as in Example 1 except an elastomeric polyurethane (ESTANE 58-238, Noveon) was used as the molten polymer. The temperature of the molten polymer was approximately 207°C and the transfer roll was at approximately 210°C. The temperature of the backup roll was approximately 38°C. A nip pressure of 175 N/lineal cm was used. The basis weight of each transferred molten polymer region was 110 grams/m². The cumulative basis weight of the transferred polymer regions on the nonwoven substrate was 151 grams/m².

### Example 10

A web was produced as in Example 1 except an elastomeric polyurethane (2103-80AE, Dow Chemical) was used as the molten polymer. The temperature of the molten polymer was approximately 210°C and the transfer roll was at approximately 210°C. The temperature of the backup roll was approximately 38°C. A nip pressure of 175 N/lineal cm was used. The basis weight of each transferred molten polymer region was 706 grams/m². The cumulative basis weight of the transferred polymer regions on the nonwoven substrate was 96 grams/m².

### Example 11

A web was produced as in Example 1 except an elastomeric polyurethane (455-203 Huntsman Chemical) was used as the molten polymer. The temperature of the molten polymer was approximately 210°C and the transfer roll was at approximately 210°C. The temperature of the backup roll was approximately 38°C. A nip pressure of 175 N/lineal cm was used. The basis weight of each transferred molten polymer region was 1265 grams/m². The cumulative basis weight of the transferred polymer regions on the nonwoven substrate was 172 grams/m².

### Example 12

A web was produced as in Example 1 except an elastomeric polyurethane (ESTANE 58-271, Noveon) was used as the molten polymer. The temperature of the molten polymer was approximately 210°C and the transfer roll was at approximately 210°C. The temperature of the backup roll was approximately 38°C. A nip pressure of 175 N/lineal cm was used. The basis weight of each transferred molten polymer region was 373 grams/m². The cumulative basis weight of the transferred polymer regions on the nonwoven substrate was 51 grams/m².

### Example 13

A web was produced as in Example 1 except an ethylene-vinylacetate copolymer (ELVAX 150, Dupont) was used as the molten polymer and a polyester spunlaced nonwoven (SONTARA 8005,40 grams/m², Dupont) was used as the substrate. The temperature of the molten polymer was approximately 189°C and the transfer roll was at approximately 191°C. The temperature of the backup roll was approximately 38°C. A nip pressure of 88 N/lineal cm was used. The basis weight of the transferred polymer was not measured.

### Example 14

A web was produced as in Example 15 except a polypropylene spunbond nonwoven (MIRATEC, 68 grams/m², PGI Nonwovens) was used as the substrate. The temperature of the molten polymer was approximately 193°C and the transfer roll was at approximately 191°C. The temperature of the backup roll was approximately 38°C. A nip pressure of 88 N/lineal cm was used. The basis weight of the transferred polymer was not measured.

### Example 15

A web was produced as in Example 1 except two different polymers were used and delivered to three separate regions on the transfer roll. The trough described in Example 1 was constructed with two dividers between the side walls so as to have three separate smaller troughs arranged in an A-B-A configuration across the transfer roll, that could receive three separate molten polymer streams. KRATON 1657 SEBS block copolymer was delivered to the 'A' troughs using the extruder described in Example 1 at a melt temperature of approximately 237°C. Polyethylene (ASPUN 6806, Dow Chemical) was delivered by a J&M Grid Melter and heated pipe to the 'B' trough at a melt temperature of approximately 218°C. The exterior surface of the transfer roll was machined using a computer controlled milling machine to have depressions in the shape of hemispheres 2.3 mm in diameter, 1.2 mm in depth, with 3.9 depressions per cm². Polyester spunlaced nonwoven (SONTARA 8005, 68 grams/m², Dupont) was used as the substrate. The transfer roll was at approximately 246°C. The temperature of the backup roll was approximately 38°C. A nip pressure of 263 N/lineal cm was used. The basis weight of each transferred molten polymer region was not measured. The cumulative basis weight of the transferred polymer regions on the nonwoven substrate was not measured.

### Example 16

To demonstrate the use of different depression geometries, a transfer roll was machined with seven different areas arranged around and across the periphery of the roll, each area having a specific depression geometry and spacing. Area 1 was machined using a computer controlled milling machine (2 mm ball diameter) to have depressions in the shape of grooves parallel to the roll axis 25 mm long, 0.75 mm in depth, 13 mm end to end spacing measured along the roll axis, 7.5 mm center to center spacing between grooves measured normal to the roll axis, with 12 rows of staggered grooves. Each row of grooves starting with a 6.4 mm shift from the previous row to create the staggered pattern. Area 2 was machined using a computer controlled milling machine (2 mm ball diameter) to have 15 rows of grooves parallel to the roll axis 114 mm long, 0.375 mm in depth, and 6.0 mm center to center spacing between grooves measured normal to the roll axis. Area 3 was machined using a computer controlled milling machine (2 mm ball diameter) to have 15 rows of grooves parallel to the roll axis 114 mm long, 0.5 mm in depth, and 6.0 mm center to center spacing between grooves measured normal to the roll axis. Area 4 was machined using a computer controlled milling machine (2 mm ball diameter) to have 12 rows of grooves parallel to the roll axis 114 mm long, 0.5 mm in depth, and 7.5 mm center to center spacing between grooves measured normal to the roll axis. Area 5 was machined using a computer controlled milling machine (2 mm ball diameter) to have 12 rows of grooves parallel to the roll axis 114 mm long, 0.875 mm in depth, and 7.5 mm center to center spacing between grooves measured normal to the roll axis. Area 6 was machined using a computer controlled milling machine (2 mm ball diameter) to have 9 rows of grooves parallel to the roll axis 114 mm long, 1.0 mm in depth, and 10.0 mm center to center spacing between grooves measured normal to the roll axis. Area 7 was machined using a computer controlled milling machine (3 mm ball diameter) to have 9 rows of grooves parallel to the roll axis 114 mm long, 0.75 mm in depth, and 10.0 mm center to center spacing between grooves measured normal to the roll axis. A web was produced as in Example 1 except a 40 mm diameter twin screw extruder fitted with a gear pump was used to deliver the molten polymer. An ultra low density polyethylene (ENGAGE 8200, DupontDow Elastomers) was used as the molten polymer and a polyester spunlaced nonwoven (SONTARA 8001, 40 grams/m², Dupont) was used as the substrate. The temperature of the molten polymer was approximately 232°C and the transfer roll was at approximately 232°C. The temperature of the backup roll was approximately 20°C.A nip pressure of 12 N/lineal cm was used. All the depressions filled and transferred well. The basis weight of the transferred polymer in the separate regions was not measured.

### Example 17

To demonstrate that a hot wire is not necessary in some instances to provide for effective transfer, a web was produced as in Example 16 except the hot wire was removed from the apparatus. The temperature of the molten polymer was approximately 232°C and the transfer roll was at approximately 232°C.. All the depressions filled and transferred well. The basis weight of the transferred polymer in the separate regions was not measured.

### Example 18

To demonstrate a multi-layer laminate, a web was produced as in Example 16 except a second nonwoven substrate (SONTARA 8001) was laminated to the first nonwoven substrate containing the transferred polymer, using a second nip at a pressure of 6 N/lineal cm. The temperature of the molten polymer was approximately 232°C and the transfer roll was at approximately 232°C. All the depressions filled and transferred well. The basis weight of the transferred polymer in the separate regions was not measured.

### Example 19

A web was produced as in Example 16 except an SEBS block copolymer (KRATON G1657, Shell Chemical) was used as the molten polymer. The temperature of the molten polymer was approximately 246°C and the transfer roll was at approximately 232°C. A nip pressure of 12 N/lineal cm was used. All the depressions filled and transferred well. The basis weight of the transferred polymer in the separate regions was not measured.

### Example 20

To demonstrate a multi-layer laminate, a web was produced as in Example 18 except KRATON G1657 was used as the molten polymer. The temperature of the molten polymer was approximately 246°C and the transfer roll was at approximately 232°C. All the depressions filled and transferred well. The basis weight of the transferred polymer in the separate regions was not measured.

### Example 21

A web was produced as in Example 16 except an elastomeric polyurethane (ESTANE 58-680, Noveon Inc.) was used as the molten polymer. The temperature of the molten polymer was approximately 210°C and the transfer roll was at approximately 210°C. A nip pressure of 12 N/lineal cm was used. All the depressions filled and transferred well. The basis weight of the transferred polymer in the separate regions was not measured.

### Example 22

To demonstrate a multi-layer laminate, a web was produced as in Example 18 except ESTANE 58-680 polyurethane was used as the molten polymer. The temperature of the molten polymer was approximately 210°C and the transfer roll was at approximately 210°C. All the depressions filled and transferred well. The basis weight of the transferred polymer in the separate regions was not measured.

### Counter Example C1

To demonstrate that some nonwovens do not have enough internal strength to provide for a good substrate, a web was produced as in Example 19 except a resin bonded polyester nonwoven (STYLE 1545, 30 grams/m², HDK Industries) was used as the substrate. The temperature of the molten polymer was approximately 246°C and the transfer roll was at approximately 232°C. A nip pressure of 12 N/lineal cm was used. After contacting the molten polymer to the nonwoven substrate in the nip, the nonwoven delaminated and transferred to the transfer roll. The adhesion of the molten polymer in the depressions to the metal of the transfer roll was greater than the internal strength of the nonwoven.

The preceding specific embodiments are illustrative of the practice of the invention. This invention may be suitably practiced in the absence of any element or item not specifically described in this document. The complete disclosures of all patents, patent applications, and publications are incorporated into this document by reference as if individually incorporated. Various modifications and alterations of this invention will become apparent to those skilled in the art without departing from the scope of this invention. It should be understood that this invention is not to be unduly limited to illustrative embodiments set forth herein.

## Claims

1. A fastening tab article comprising:
a substrate comprising first and second major surfaces, an elongation axis, and a distal end;
and one or more elastic elements attached to the substrate, wherein the one or more elastic elements comprise discrete elastomeric thermoplastic regions, wherein the one or more elastic elements comprise a length greater than a width, and wherein the length of the one or more elastic elements is substantially aligned with the elongation axis, and further wherein the combined amount of elastomeric thermoplastic in the one or more elastic elements changes when moving in one direction along the elongation axis.

2. The fastening tab of claim 1 further comprising one or more bonding sites on the fastening tab.

3. The fastening tab of claim 1 further comprising one or more mechanical fasteners attached to the first major surface of the substrate.

4. An article according to claim 3, wherein each mechanical fastener of the one or more mechanical fasteners comprises a discrete thermoplastic region wherein at least a portion of at least one mechanical fastener infiltrates the first major surface of the substrate.

5. An article according to claim 3 or 4, wherein each mechanical fastener of the one or more mechanical fasteners further comprises a plurality of fastening structures located thereon, the fastening structures facing away from the first major surface of the substrate.

6. An article according to any of claims 1-5, wherein the width of the one or more elastic elements changes when moving in one direction along the elongation axis.

7. An article according to any of claims 1-6, wherein the thickness of one or more elastic elements changes when moving in one direction along the elongation axis.

8. An article according to any of claims 1-7, wherein two or more elastic elements have different lengths in one direction along the elongation axis.

9. An article according to any of claims 1-8, wherein the amount of elastomeric thermoplastic material in each elastic element of the one or more elastic elements increases when moving away from the distal end along the elongation axis.

10. An article according to any of claims 1-5, wherein the amount of elastic thermoplastic material in each elastic element of the one or more elastic elements is constant when moving in one direction along the elongation axis, and wherein the change in the amount of elastic thermoplastic is provided by a change in the number of elastic elements when moving in the one direction along the elongation axis.

11. An article according to any of claims 1-10, wherein a portion of the elastomeric thermoplastic of each elastic element of the one or more elastic elements infiltrates a portion of the substrate.

12. An article according to any of claims 1-11, wherein each elastic element of the one or more elastic elements is located between the substrate and a second substrate.

13. An article according to any of claims 1-11, wherein at least one elastic element of the one or more elastic elements is located on the first major surface or the second major surface of the substrate.

14. An article according to any of claims 2 and 6-13, wherein the one or more bonding sites comprise adhesive.

15. An article according to any of claims 2 and 6-13, wherein the one or more bonding sites comprise cohesive material.

16. An article according to any of claims 2 and 6-14, wherein the one or more bonding sites comprise mechanical fasteners comprising a plurality of fastening structures located thereon.

17. An article according to any of claims 1-16, wherein the fastening tab article is attached to a garment.

18. A method for producing a composite web, the method comprising:
providing a transfer roll comprising an exterior surface that comprises one or more depressions formed therein;
delivering a molten elastomeric thermoplastic composition onto the exterior surface of the transfer roll;
wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one or more depressions after wiping the molten elastomeric thermoplastic composition from the exterior surface of the transfer roll; and
transferring at least a portion of the molten elastomeric thermoplastic composition in the one or more depressions to a first major surface of a substrate by contacting the first major surface of the substrate to the exterior surface of the transfer roll and the molten elastomeric thermoplastic composition in the one or more depressions, followed by separating the substrate from the transfer roll, wherein one or more discrete polymeric regions comprising the elastomeric thermoplastic composition are located on the first major surface of the substrate after separating the substrate from the transfer roll.

19. A method for producing a composite web, the method comprising:
providing a transfer roll comprising an exterior surface that comprises one or more depressions formed therein;
delivering a molten elastomeric thermoplastic composition onto the exterior surface of the transfer roll;
wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one

20. A method for producing a composite web, the method comprising:
providing a transfer roll comprising an exterior surface that comprises one or more depressions formed therein;
delivering a molten elastomeric thermoplastic composition onto the exterior surface of the transfer roll;
wiping the molten elastomeric thermoplastic composition from the exterior surface of the transfer roll, wherein a portion of the molten elastomeric thermoplastic composition enters the one or more depressions, and further wherein the portion of the molten elastomeric thermoplastic composition in the one or more depressions remains in the one or more depressions after wiping the molten elastomeric thermoplastic composition from the exterior surface of the transfer roll;
transferring at least a portion of the molten elastomeric thermoplastic composition in the one or more depressions to a first major surface of a first substrate by contacting the first major surface of the first substrate to the exterior surface of the transfer roll and the molten elastomeric thermoplastic composition in the one or more depressions, followed by separating the first substrate from the transfer roll, wherein one or more discrete polymeric regions comprising the elastomeric thermoplastic composition are located on the first major surface of the first substrate after separating the first substrate from the transfer roll; and
laminating a second substrate to the first major surface of the first substrate, wherein the one or more discrete polymeric regions on the first substrate are located between the first substrate and the second substrate after laminating the second substrate to the first substrate.

21. A method for producing a composite web, the method comprising:
providing a first substrate comprising a first major surface and a second major surface, a plurality of discrete elastomeric polymeric regions formed of an elastomeric thermoplastic composition located on the first major surface of the first substrate, wherein each discrete elastomeric polymeric region of the plurality of discrete elastomeric polymeric regions infiltrates the first major surface of the first substrate;
providing a second substrate comprising a first major surface and a second major surface, a plurality of discrete polymeric regions formed of a thermoplastic composition located on the first major surface of the second substrate, wherein each discrete polymeric region of the plurality of discrete polymeric regions infiltrates the first major surface of the second substrate; and
laminating the first substrate to the second substrate.

22. An elastic fastening article comprising:
a substrate comprising first and second major surfaces;
one or more mechanical fasteners attached to the first major surface of the substrate, wherein each mechanical fastener of the one or more mechanical fasteners comprises a discrete thermoplastic region infiltrating the first major surface of the substrate, and wherein each mechanical fastener of the one or more mechanical fasteners further comprises a plurality of fastening structures located thereon, the fastening structures facing away from the first major surface of the substrate; and
one or more elastic elements attached to the substrate, wherein each elastic element of the one or more elastic elements comprises a discrete elastomeric thermoplastic region infiltrating a portion of the substrate.

23. An elastic article comprising:
a substrate comprising first and second major surfaces;
one or more elastic elements attached to the substrate, wherein each elastic element of the one or more elastic elements comprises a discrete elastomeric thermoplastic region infiltrating a portion of the substrate; and
one or more bonding sites located on the first major surface of the substrate.

24. An elastic article comprising:
a substrate comprising first and second major surfaces;
one or more elastic elements attached to the substrate, wherein each elastic element of the one or more elastic elements comprises a discrete elastomeric thermoplastic region infiltrating a portion of the substrate; and
one or more slits formed through the substrate, wherein at least one of the one or more elastic elements spans each slit of the one or more slits.

25. An elastic article comprising:
a substrate comprising first and second major surfaces;
one or more elastic elements attached to the substrate, wherein each elastic element of the one or more elastic elements comprises a discrete elastomeric thermoplastic region infiltrating a portion of the substrate; and
one or more pleats formed in the substrate, wherein at least one of the one or more elastic elements spans at least one pleat of the one or more pleats.
